# EUROPEAN PATENT SPECIFICATION

(11) **EP 1 963 491 B1**
(45) Date of publication and mention of the grant of the patent: **12.01.2011**
(21) Application number: 06848082.1
(22) Date of filing: 21.12.2006
(51) Int. Cl.: C12N 5/0784, G01N 33/50

(54) **IN VITRO METHOD OF EVALUATING THE POTENTIAL REACTION OF AN ANIMAL TO AN AGENT**
IIN VITRO METHODE UM EINE POTENTIELLE REAKTION EINES TIERES AN EINEN AGENTEN ZU BESTIMMEN
METHODE IN VITRO POUR EVALUER LA REACTION POTENTIELLE D'UN ANIMAL A UN AGENT

(30) Priority: 21.12.2005 US 752033 P
(43) Date of publication of application: 03.09.2008
(60) Divisional application: 10151003.0
(73) Proprietor: VaxDesign Corporation, Orlando, FL 32826 (US)
(72) Inventor: DRAKE, Donald, III, Orlando, FL 32804 (US); MOE, David, Orlando, FL 32810 (US); LI, Conan, San Jose, CA 95129 (US); FAHLENKAMP, Heather, Cleveland, Oklahoma 74020 (US); SANCHEZ-SCHMITZ, Guzman, Orlando, FL 32825 (US); HIGBEE, Russell, Orlando, FL 32825 (US); PARKHILL, Robert, Orlando, FL 32828 (US); WARREN, William, L., Orlando, FL 32828 (US)
(74) Representative: MacLean, Martin Robert
(86) International application number: PCT/US2006/049128
(87) International publication number: WO 2007/076061

(56) References cited:
- WO-A-03/050271
- QU CHUNFENG ET AL: "Autocrine type I IFN and contact with endothelium promote the presentation of influenza A virus by monocyte-derived APC." JOURNAL OF IMMUNOLOGY, vol. 170, no. 2, 15 January 2003 (2003-01-15), pages 1010-1018, XP002432204 ISSN: 0022-1767 cited in the application
- RANDOLPH G J ET AL: "Differentiation of monocytes into dendritic cells in a model of transendothelial trafficking" SCIENCE 16 OCT 1998 UNITED STATES, vol. 282, no. 5388, 16 October 1998 (1998-10-16), pages 480-483, XP002432205 ISSN: 0036-8075 cited in the application
- SEGUIN ROSANNE ET AL: "Human brain endothelial cells supply support for monocyte immunoregulatory functions." JOURNAL OF NEUROIMMUNOLOGY, vol. 135, no. 1-2, February 2003 (2003-02), pages 96-106, XP002432206 ISSN: 0165-5728 cited in the application
- MANNA P P ET AL: "Differentiation and functional maturation of human CD14<+> adherent peripheral blood monocytes by xenogeneic endothelial cells: Up-regulation of costimulation, cytokine generation, and toll-like receptors" TRANSPLANTATION 27 JUL 2002 UNITED STATES, vol. 74, no. 2, 27 July 2002 (2002-07-27), pages 243-252, XP009083121 ISSN: 0041-1337

## Description

### BACKGROUND OF THE INVENTION

The generation of protective immunity against pathogens and tumors in mammals requires specialized cells that can present foreign or altered self antigens to T cells. Dendritic cells (DCs) are thought to be the most potent of these antigen-presenting cells (APCs) because they efficiently acquire and process antigen for presentation in major histocompatibility complex (MHC) molecules and express high levels of T cell costimulatory ligands, both of which are necessary to trigger complete differentiation of naïve T cells into competent effector cells. It is also thought that DCs are more capable than other APCs of cross-presenting exogenous proteins through the endogenous (MHC class I) pathway, making them particularly important for generating cytotoxic T lymphocyte responses against tumors and extracellular pathogens.

Dendritic cells are typically found in most tissues of the body and are derived from circulating monocytes that traverse the vascular endothelium into peripheral tissues. Under normal conditions, these cells have a high capacity for antigen acquisition, but low levels of surface MHC and costimulatory molecule expression.

Injury or infection triggers a marked increase in the number of DCs at the affected site. Additionally, these DCs acquire an activated phenotype, characterized by increased expression of soluble and membrane-bound molecules, decreased capacity to acquire antigen, and enhanced migration towards secondary lymphoid tissues. In lymph nodes, these cells are potent stimulators of antigen-specific T cell activation. For a more complete synopsis on the biology of DCs, see the recent review by Rossi & Young (J Immunol 175:1373-1381 (2005).

It is beneficial to construct a wholly *in vitro* immune response for screening and assessing the immunogenicity of vaccines, drugs, or other compounds. Employing human subjects for this purpose may be dangerous and is costly, while using laboratory animals can lead to results that do not accurately reflect the response in humans.

Until now, there has been no convenient, cost effective, and automatable in *vitro* technique for preparing DCs from peripheral blood cells in a manner that simulates what occurs in the body. Monocytes can be segregated from peripheral blood by antibody separation (*e.g*.; magnetic beads), but this is cumbersome and costly, because it involves the use of specialized antibodies directed against the cells of interest. Those monocytes must then be further differentiated with exogenous factors, such as IL-4 and GM-CSF (Romani et al. (1994) J Exp Med 180:83-93; Sallusto & Lanzavecchia (1994) J Exp Med 179:1109-1118), which may lead to DCs that do not necessarily mimic those involved in an *in vivo* immune response (Thurnher et al. (2001) FASEB J 15:1054-1061). Peripheral blood monocytes that transmigrate through an endothelial cell layer that is atop a collagen substrate have been shown to differentiate into functional DCs (Qu et al. (2003) J Immunol 170: 1010-1018; Randolph et al. (1998) Science 282:480-483).

The generation of protective immunity against infection and tumors requires specialized cells that can present foreign or altered self antigens to T cells. While several cell types can act as APCs, DCs are the most potent of these and the only ones capable of inducing CD4⁺ and CD8⁺ T cell responses against naïve antigens. Under normal conditions, immature DCs (iDCs) actively acquire antigen via various pathways of endocytosis, but express low levels of surface major histocompatibility complex (MHC) and T cell costimulatory molecules. An encounter with inflammatory signals or common pathogen motifs (Toll-like receptor ligands) triggers a maturation program in DCs that lessens their ability to uptake exogenous proteins, increases their surface expression of MHC/peptide complexes and ligands important for T cell activation, and enhances their migration towards secondary lymphoid tissues (Rossi & Young (2005) J Immunol 175, 1373-1381). It is these matured, antigen-loaded DCs that are particularly well-suited for inducing primary T cell responses within secondary lymphoid tissues.

Tissue-resident DCs comprise a heterogeneous population of cells that is found in most organs of the body. Short-lived circulating monocytes, which give rise to iDCs, traverse the vascular endothelium into peripheral tissues in a constitutive manner, though infection or injury triggers an increased accumulation of these cells at the inflamed site. Within tissues, a subset of the extravasated monocytes differentiate into iDCs, with the milieu of the local microenvironment often influencing the phenotype and functional activity of APCs residing in a particular site. For example, gut-associated DCs populate Peyer's patches, where they receive antigens from M cells and act as the resident APCs of mucosal tissue. Langerhans cells, on the other hand, are found primarily in the skin and play a key role in the induction of adaptive responses following infection.

Several laboratories have worked to develop *in vitro* systems which recapitulate the cell interactions and signaling pathways that trigger monocyte to DC differentiation in vivo. For instance, the groups of Muller and Randolph (Qu et al. (2003) J Immunol 170, 1010-1018; Randolph et al. (1998) Science 282, 480-483) pioneered the development of tissue constructs that utilize HUVECs grown on a support matrix to promote the generation of human DCs from blood monocytes that have transmigrated through the endothelial layer. The APCs derived from this system resembled DCs in phenotype and ability to trigger allogeneic and primary antigen-specific T cell responses (Qu et al. (2003) J Immunol 170, 1010-1018; Randolph et al. (1998) Science 282, 480-483). While this tissue model might generated APCs that more accurately represent DC populations found *in vivo,* its complexity makes it impractical for widespread use. In another approach, adherent monocytes cocultured directly with human or porcine endothelial cells gave rise to potent APCs that produced proinflammatory cytokines, expressed high levels of costimulatory ligands, and efficiently stimulated allogeneic T cells. A limitation of this technique is that the DCs had to be selected from contaminating endothelial cells by magnetic bead selection before any functional analyses could be performed.

There has been tremendous interest in better understanding the biology of DCs because of their specialized role in orchestrating primary cellular and humoral immune responses. The paucity of DCs in the body, combined with the limited availability of tissues samples from humans, make it difficult to evaluate these cells in an *ex vivo* manner. As a result, the study of cytokine-derived DCs, *i.e.,* purified blood monocytes that have been cultured in exogenous growth factors (GM-CSF and IL-4), has contributed great insight into this unique cell population and provided a source of APC for clinical applications. The utility of cytokine-derived DCs is limited, however, because this culture method fails to replicate the physiology involved in the development of DCs from circulating monocytes in the body. Additionally, some researchers have suggested that this DC population lacks full APC functionality and may not accurately represent DC populations found under physiologic conditions (Romani el al. (1994) J Exp Med 180:83-93 ; Sallusto & Lanzavecchia (1994) J Exp Med 179,1109-1118; Thurnher et al. (2001) FASEB J. 15, 1054-1061).

The present invention provides an *in vitro* method of evaluating the potential reaction of an animal to an agent as described in the claims.

The present specification describes a method for generating large numbers of dendritic cells comprising:
- culturing endothelial cells on top of a porous membrane, wherein said membrane is housed in an upper chamber of a well that is suspended over, and is separable from, a lower chamber of a well:
- applying peripheral blood mononuclear cells (PBMCs) to the endothelial cells on the porous membrane;
- at least about 48 hours after application of the PBMCs, removing the upper chamber of the well, housing the porous membrane and endothelial cells; and
- isolating dendritic cells from the lower chamber of the well.

The present invention also provides a method of evaluating the potential reaction of an animal to an agent, said method comprising:
- producing a first well comprising:
   - a first porous membrane as the base;
   - a ECM material affixed on top of said first porous membrane; and
   - a second porous membrane affixed on top of said ECM material;
- inverting said first well into a second well comprising cell media;
- culturing endothelial cells on bottom of said first porous membrane;
- applying peripheral blood mononuclear cells (PBMCs) to the endothelial cells;
- after ~1.5 hours washing said PBMCs and said endothelial cells off of the bottom of said first porous membrane, wherein dendritic cells are now present in said ECM material;
- removing said first well from said second well comprising cell media and placing said first well with said second porous membrane facing up into a third well comprising as its base a three-dimensional artificial lymphoid tissue, comprising a second ECM material and a plurality of lymphocytes and leukocytes;
- applying an agent to the top of said second porous membrane, allowing the dendritic cells to migrate out of said first ECM material and into said three-dimensional artificial lymphoid tissue; and
- evaluating the immune response to said agent.

The present specification describes a method for generating large numbers of dendritic cells comprising:
- producing a first well comprising:
   - a first porous membrane as the base;
   - endothelial cells cultured on the bottom of said first porous membrane;
   - a second porous membrane situated above, and separated from, said first porous membrane;
   - endothelial cells cultured on the top of said second porous membrane; and
   - cell culture media comprising an agent located between said first porous membrane and said second porous membrane;
- inverting said first well into a second well comprising cell media;
- applying peripheral blood mononuclear cells (PBMCs) to the endothelial cells cultured on the top of said second porous membrane;
- at least about 48 hours after application of the PBMCs, removing said first well from said second well; and
- isolating dendritic cells from said second well.

### BRIEF DESCRIPTION OF THE FIGURES

**Figure 1****.** Schematic diagram of an embodiment of the invention, using a Transwell^{®} device. HUVECs are grown to confluency on Transwell^{®} membranes and then total PBMC are applied to the upper chamber for ~1.5 h (step 1). Unbound cells are washed away and the remaining leukocytes are allowed to transmigrate for ~48 h. The Transwell^{®} is removed and DCs are then collected for analysis or pulsed with antigen for an additional ~2 days (step 2).

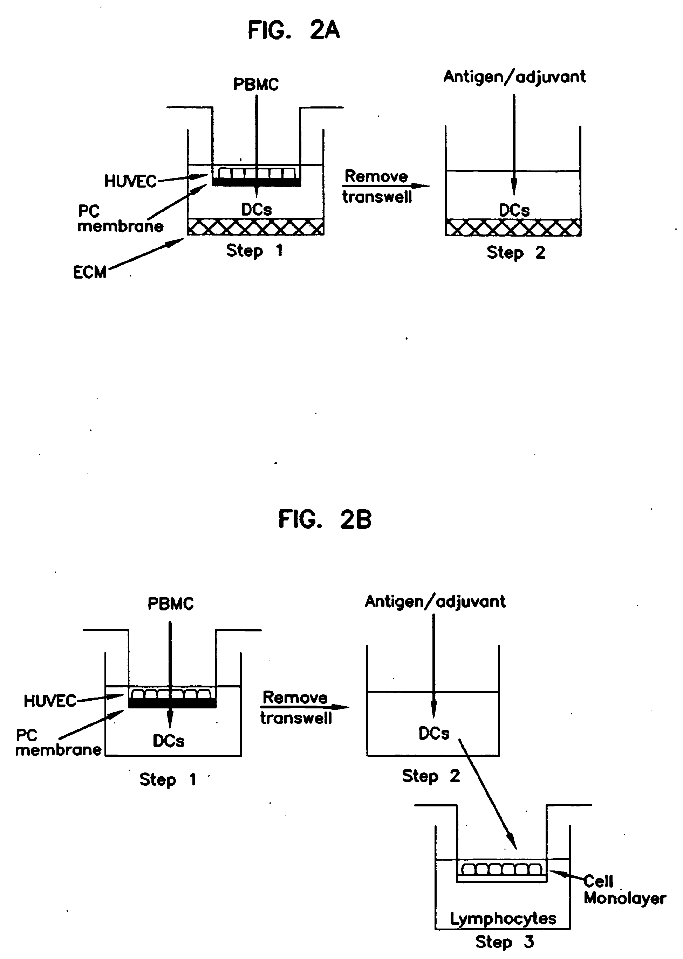

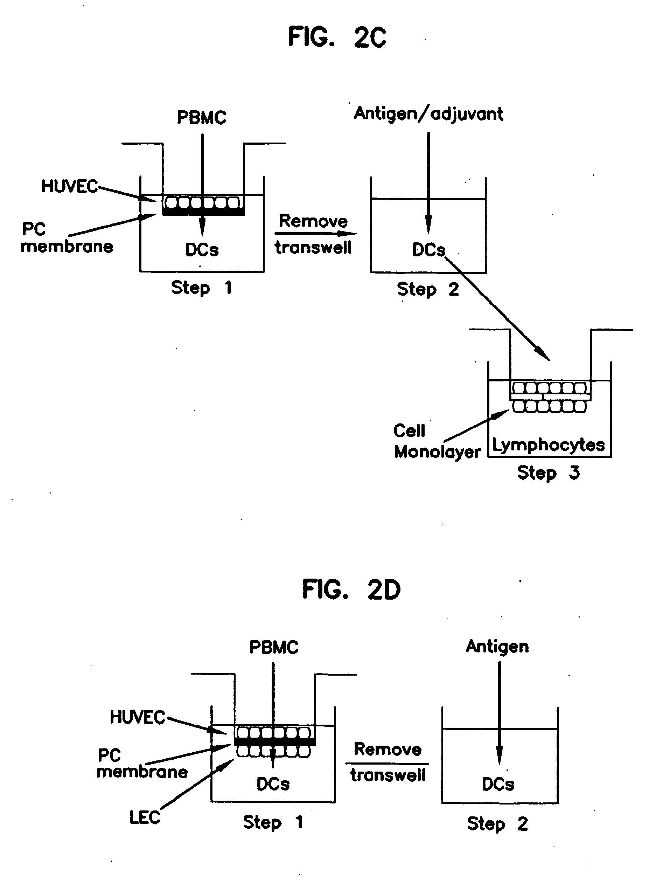

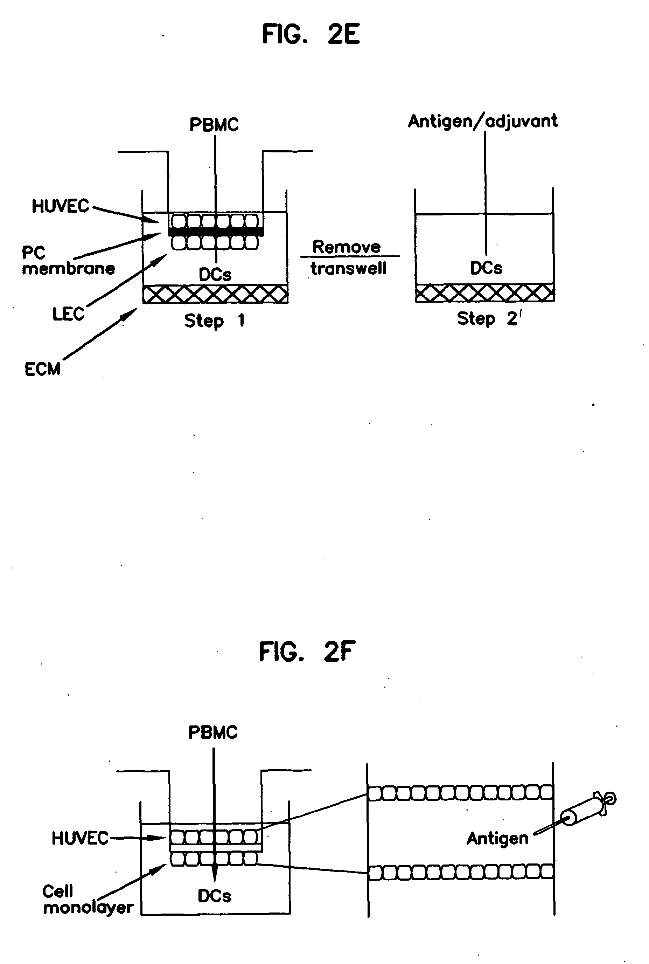

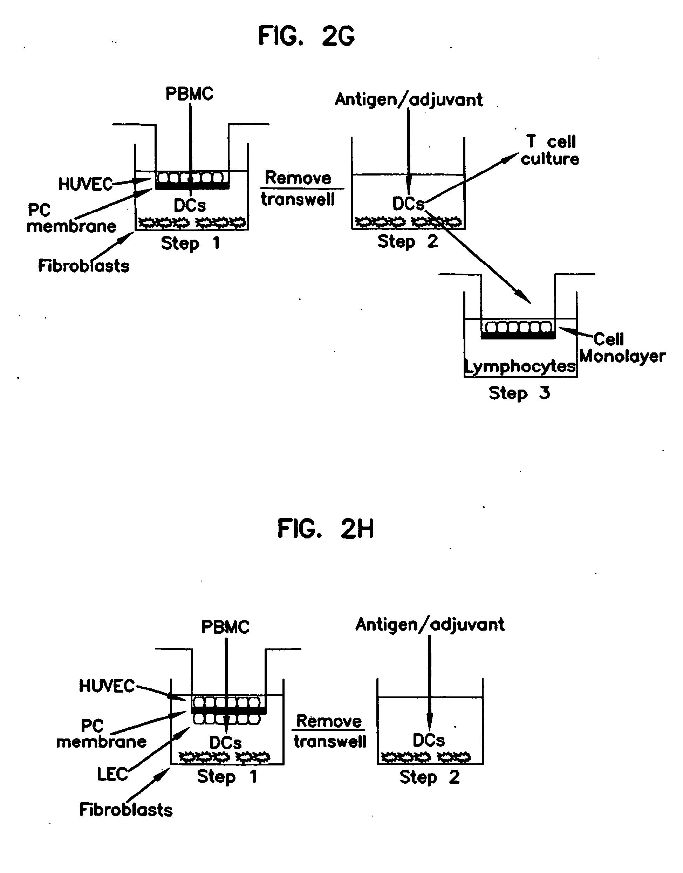

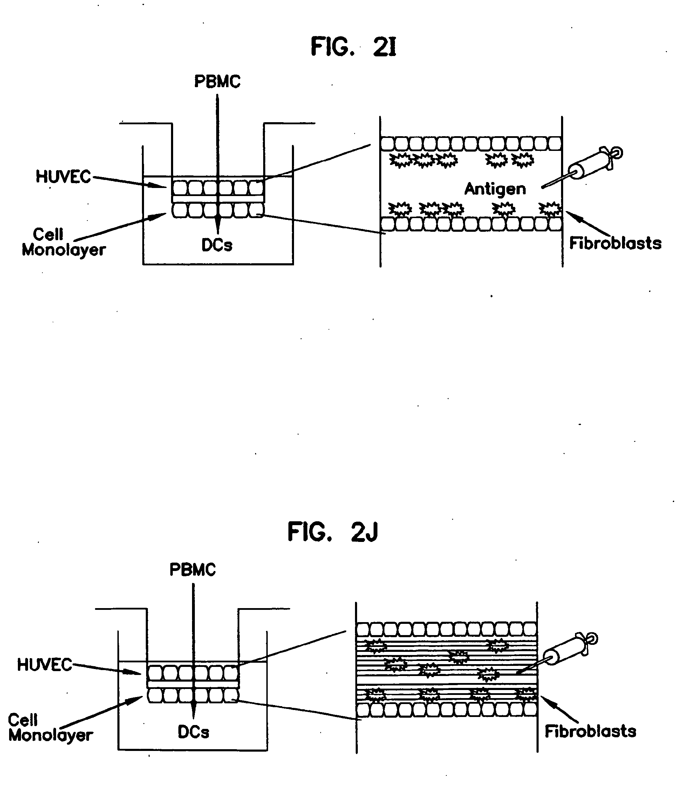

**Figure 2****.** The complexity of the membrane device can be increased by, for example, the inclusion of secondary cell populations, ECM materials and additional membrane layers. Monocytes that traverse through an endothelial monolayer can contact ECM in the lower chamber of the membrane device (A). Two membrane devices can be used to mimic the normal pathway of monocyte migration from the blood into the tissue (through the HUVECs) and from the tissue into the lymphatics (through a second cell layer, such as, for example, lymphatic endothelial cells). The second monolayer can be cultured on the upper (B) or lower (C) side of the membrane device, mimicking transmigration or reverse transmigration, respectively. The membrane can be coated on both sides with the same or different cell types (D); ECM can also be incorporated into the lower chamber with this design,(E). A modified Transwell^{®} can be constructed that contains a central chamber sandwiched between two membranes/cell monolayers (F). Fibroblasts or other cells types that are important in DC differentiation or antigen-presenting activity can be included in the lower chamber of a single membrane device (G or H) or in the middle of a dual membrane device (1 and J). ECM can also be incorporated into the dual-membrane device (H).

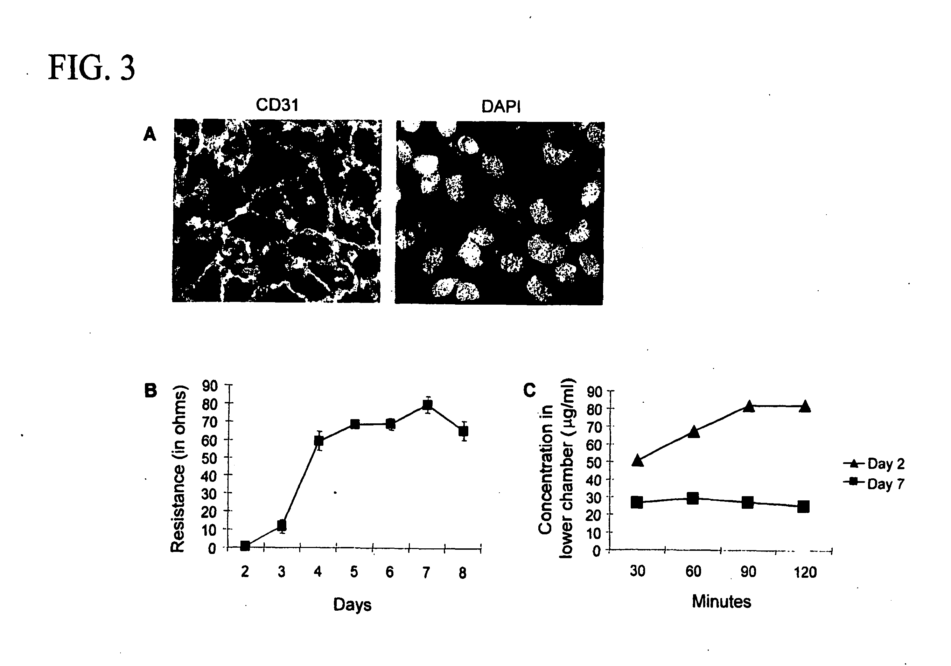

**Figure 3****.** HUVECs form confluent monolayers on Transwell^{®} membranes. Primary HUVECs were seeded in the upper chamber of Transwell^{®} s and analyzed for confluency and the formation of tight-gap junctions. (A) On day 7 after seeding, the cells were fixed, surface-labeled with an antibody specific for CD31, and the nuclei were stained with DAPI. (B) At the indicated time points, electrical resistance (TEER) readings were collected and normalized against the values for empty Transwell^{®}s on the same day. The error bars represent 1 SD of triplicate readings in each well. (C) Diffusion through the endothelial layer was measured with a 70kDa FITC-dextran conjugate at the indicated time points.

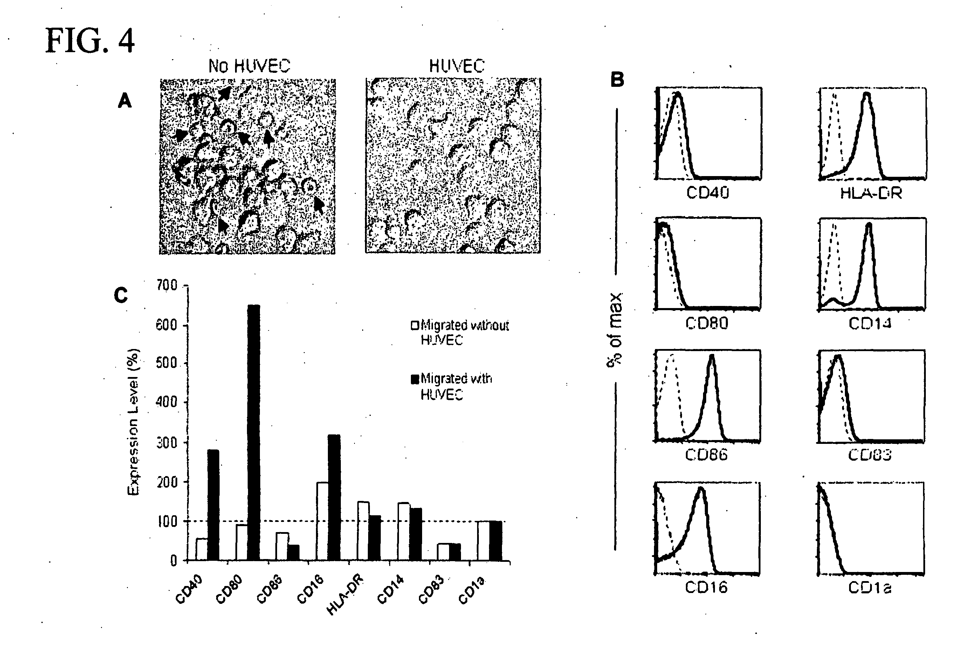

**Figure 4****.** Monocyte transmigration through an endothelial monolayer is sufficient to trigger their differentiation towards a DC phenotype. (A) Cells that passed through a PC membrane in the absence (left) or presence (right) of a HUVEC monolayer were imaged by phase microscopy (20 × objective). Arrows indicate contaminating red blood cells or lymphocytes. (B) CD14-purified monocytes (non-transmigrated) were put into culture and then labeled with monoclonal antibodies specific for the indicated markers ~2 d later. The dotted line indicates background fluorescence with the appropriate isotype control. (C) Cells that transmigrated through the membrane in the absence and presence of a HUVEC monolayer were also examined for expression of the indicated surface proteins ~2 d after PBMC were applied to the Transwell^{®}. The expression level of migrated monocytes is plotted as a percent increase or decrease over the MFI on non-migrated monocytes, which are set to 100%. All analysis plots are gated on monocytes only.

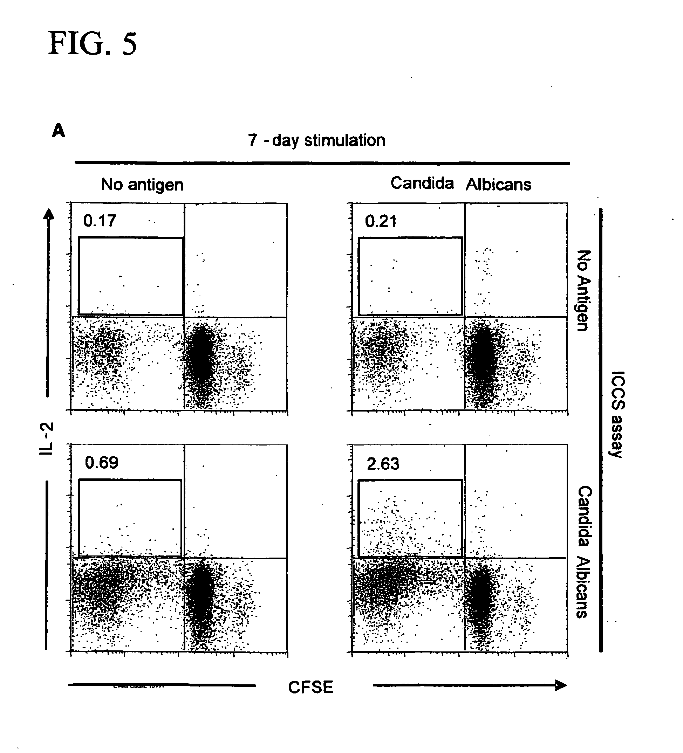

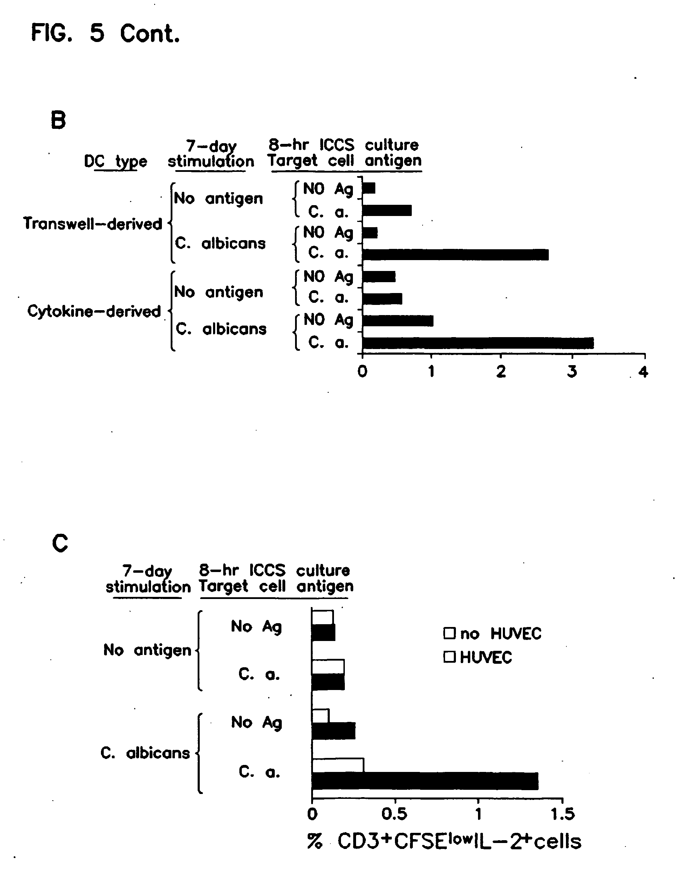

**Figure 5****.** Transwell^{®}-derived DC are potent stimulators of antigen-specific T cell responses. Transwell^{®}-derived DC were pulsed with antigen and cultured at a ~1:20 ratio with autologous T cells that had been labeled with CFSE. About 7 d later, the T cells were restimulated with autologous antigen-pulsed DC (~1:10 ratio to T cells) for -8 h and then assayed for IL-2 production by ICCS. Unpulsed DC were included as a negative control. (A) Dot plots showing representative CFSE and IL-2 staining patterns. The capacity of Transwell^{®}- and cytokine-derived DCs to stimulate recall *C*. *albicans-*specific T cell responses were compared in (B), while transmigrated cells from HUVEC-negative and -positive Transwell^{®}s served as APCs in (C). In both assays, the T cells were analyzed for cytokine production by flow cytometry and the graph shows the frequency of lymphocyte-gated CD3⁺CFSEl^{low}IL-2⁺ cells. Different donors were used in each assay.

**Figure 6****.** Example configurations of the vaccination site.

**Figure 7****.** An example of laser-micromachined polycarbonate (PC) membranes in a Transwell^{®}-based model and an outline of the process of casting collagen in a well-based model.

**Figure 8****.** Cell migration within the collagen membrane and transmigrated cells on the bottom of the plate.

**Figure 9****.** Cell migration and reverse transmigration.

**Figure 10****.** Levels of expression of HLA-DR in the migrated cells each model.

**Figure 11****.** Levels of expression of CD86 in the migrated cells each model.

**Figure 12****.** Levels of expression of CCR7 in the migrated cells each model.

**Figure 13****.** Building-in complexity to the VS model.

**Figure 14****.** Antigen introduction into the VS model. The flipping collagen membrane model as an example.

**Figure 15****.** Adherent transmigrated monocytes phenotypically resemble macrophages. PBMCs were applied to the upper chamber of a Transwell^{®} containing HUVEC and at ~48 h the migrated, non-adherent and adherent cells were collected from the lower chamber. The cells were labeled with specific antibodies and analyzed by flow cytometry. The MFI for each marker on adherent and non-adherent cells are represented graphically.

**Figure 16****.** Transmigrated APC have phagocytic activity. The non-adherent transmigrated APC were harvested from Transwell^{®}s and incubated with FITC-labeled dextran beads (~1 µm) or zymosan particles, in the absence (thin line) or presence (thick line) of 20 µg/mL cytochalasin D, for -24 h. The cells were analyzed by flow cytometry in the presence of trypan blue, which quenches any extracellular FITC fluorescence. This ensures that the only signal detected originates from material within the cell.

**Figure 17****.** Transmigratory DCs respond to maturation stimuli. ~2 d after PBMC application to the Transwell^{®}s, the transmigrated cells were harvested and incubated for an additional -48 h in the absence or presence of TNF-α and LPS. Thereafter, the cells were incubated with the antibodies specific for the indicated markers and analyzed by flow cytometry. Thin solid lines = non-matured cells; Thick solid line = matured cells; dotted lines = isotype controls. All analysis plots include only gated monocytes.

**Figure 18****.** A transformed endothelial cell line can he used to trigger monocytes differentiation to DCs in the Transwell^{®} system. The ability of Transwell^{®}-derived APC from cultures containing primary and transformed HUVEC were compared, as described in Fig. 5, using PBMC from a single donor. This data is representative of at least 3 experiments.

### DESCRIPTION OF THE INVENTION

Embodiments of the present invention are described in the claims. The present specification describes invention a simple and convenient Transwell^{®}-based culture method for the endothelial cell-mediated differentiation of DCs from blood monocytes. This system produces DCs with a frequency and purity comparable to more traditional culture methods for culturing DCs *in vitro,* but does so in only about two days, in the absence of exogenous factors and without the need for a tissue construct containing a support matrix. The transmigrated APCs derived from these cultures resemble classical *in vitro* DCs in their expression of MHC and costimulatory molecules and capacity to induce antigen-specific T cell responses. The use of a durable and fast-growing transformed endothelial cell line, which yields APCs that are comparable to those obtained when primary endothelial cells were used in the system, makes this approach a highly convenient are reliable technique for the generation of highly purified DCs.

Human dendritic cells (DCs) for research and clinical applications are typically derived from purified blood monocytes that are cultured in a cocktail of cytokines for a week or more. Because it has been suggested that these cytokine-derived DCs may be deficient in some important immunological functions and might not accurately represent antigen-presenting cell (APC) populations found under physiologic conditions, there is a continuing need for methods that allow for the generation of DCs in a more physiologically relevant manner. Previous studies have demonstrated that endothelial cells can be used to promote the differentiation of monocytes into DCs.

The specification describes a simple and reliable method for generating large numbers of highly purified DCs that is based on a single migration of, for example, human blood monocytes through, for example, human umbilical vein endothelial cells (HUVECs) that are cultured in, for example, a Transwell^{®} device, or another similar device. The resultant APC₃, harvested from the lower Transwell^{®} chamber, resemble other *in vitro*-generated DC populations in their expression of major histocompatibility (MHC) and costimulatory molecules, ability to phagocytose foreign antigens, and capacity to trigger antigen-specific T cell responses. A fast growing, transformed endothelial cell line may be used, instead of primary HUVECs, to trigger the differentiation of monocytes into iDCs.

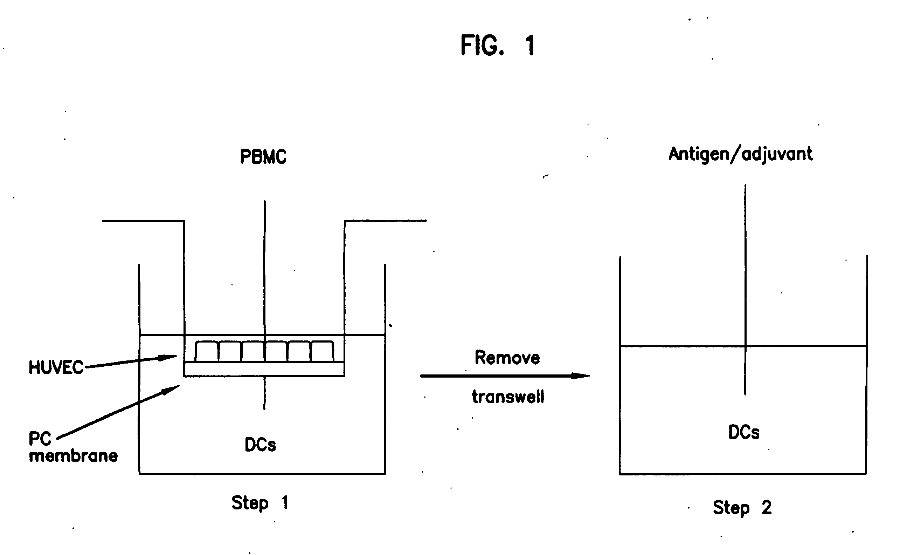

The specification describes a novel approach for the endothelial-driven development of human DCs from blood monocytes in the absence of exogenous factors. Figure 1 provides a diagrammatic representation of the method, which starts with a layer of endothelial cells being grown to confluency in, for example, a Transwell^{®} chamber. A non-immunogenic and biologically inert polycarbonate (PC) membrane, with, for example, ~1-5 µm pores, preferably ~5 µm pores that permit cell transmigration, provides support for the growth of HUVEC. The membrane is housed in an upper chamber that is suspended over, and is separable from, a lower chamber (tissue culture well). When whole PBMCs are applied to the upper chamber, the endothelial cells permit the selective passage of monocytes through the membrane and concomitantly regulate and promote their differentiation into DCs. About two days after the Transwell^{®} is seeded with PBMCs, the upper chamber is removed and antigen, in the presence or absence of additional maturation stimuli; is added to the DCs in the lower chamber.

This technique offers several advantages over the current methods of *in vitro* cytokine-driven DC development, including:
- the rapidity of this approach, with DC differentiation occuring in only about two days,
- the differentiation process itself, which is more akin to the development of DCs under physiologic conditions,
- the cost-effectiveness of the system, because no monocyte pre-selection is • necessary and DC development occurs in the absence of expensive recombinant cytokines.

The specification describes method using endothelial cells cells to drive the development of DCs in about two days, in the absence of any exogenous growth factors and without the pre-selection of monocytes from bulk PBMC. This method loosely replicates the process of blood monocyte extravasation through vessel walls, allows the generation of a highly purified APC population that resemble classical DCs in morphology, phenotype, and function.

While others have developed tissue constructs to generate human *in vitro* DCs in a related manner (Qu et al. (2003) J Immunol 170, 1010-1018 ; Randolph et al, (1998) Science 282, 480-483), the methods described in the present specification are unique in their simplicity. No-3-dimensional support matrix is required for the culture of endothelial cells, as has been used previously, and unlike other methods, the present method is amenable to the use of fast-growing transformed endothelial cell lines, which are advantageous compared to primary HUVEC because of their consistency and rapid growth rates.

Circulating monocytes can differentiate into either iDCs or macrophages once they traverse the vasculature into tissues. The tissue construct described here supports the differentiation of blood monocytes into both cell types; cells that reverse-transmigrate out of the subendothelial collagen resemble iDCs, whereas macrophages remain in the extracellular matrix (Qu et al, (2003) J lmmunol 170, 1010-1018 ; Randolph et a/. (1998) Science 282, 480-483). The geometry of the Transwell^{®} device, with monocytes traversing a confluent endothelial layer in the upper chamber, is such that both subpopulations are collected in the lower chamber. Conveniently, the non-adherent/loosely adherent iDCs are readily isolated from the strongly adherent macrophages by gently washing the wells with warm media; this approach yields 90% pure DCs (data not shown). ~ 100×10⁶ PBMC applied to the Transwell^{®}-endothelial cell system yields ~5 × 10⁶ non-adherent iDCs, which is comparable to the ~4×10⁶ iDCₛ that can be expected when monocytes are purified from the same number of PBMC and cultured in exogenous cytokines for ~7 days (data not shown).

A key role for endothelial cells in promoting the differentiation of monocytes to DCs in this Transwell^{®}-based system was highlighted by the finding that cells having transmigrated through a PC membrane in the absence of HUVEC layer were similar to non-migrated cells in their surface marker profile and ability to trigger antigen-specific T cell responses. These results are consistent with a previous observation that monocytes having contacted HUVECs were more adept than unmanipulated monocytes at stimulating T cell activity (Qu et al. (2003) J Immunol 170, 1010-1018 ; Randolph et al. (1998) Science 282, 480-483). Previous studies have suggested that this differentiation is promoted by direct cell-cell contact between the endothelial cells and monocytes, though the specific interactions mediating this differentiation program remain undefined. Although our results on the use of endothelial cell-layered porous membranes to promote the development of DCs may differ with other reports in the literature, the disparity in the results are likely easily explained by differences in the model systems. For instance, Seguin et al. observed that monocytes transmigrating through an endothelial cell layer on a porous membrane were actually worse than non-migrated monocytes in APC functionality, but these results were obtained with brain-derived endothelial cells (Seguin et al. (2003) J Neuroimmunol 135, 96-106).

Because DCs are a heterogeneous population, with phenotypes that are reflective of the tissue microenvironment in which they are found, it has thus far been difficult to identify a single marker that is common to all DC populations. For this reason, it is important to use several criteria to accurately discriminate DCs from other cell types. The non-adherent APCs harvested from the Transwell^{®} system had many of the functional attributes that are characteristic of DCs derived from other *in vivo* and *in vitro* sources. For instance, the cells had long processes, or dendrites, extending from the cell body (data not shown), which have been shown to aid antigen presentation by increasing the surface area of the cell. As well, they efficiently acquired antigen, as demonstrated by their ability to phagocytose latex beads and yeast particles, and were equal to cytokine-derived DCs in their ability to trigger functional T cell responses against recall antigens. This latter feature of the Transwell^{®}-derived APCs provides the most compelling argument that these cells are indeed DCs, as no other APC population is capable of stimulating the proliferation and differentiation of T cells into competent effectors (Rossi & Young (2005) J lmmunol 175, 1373-1381).

While the Transwell^{®}-derived APC had all the functional traits of DCs, they expressed a unique surface profile that differed from other *in vitro* DC populations. Cytokine-derived human DCs (*i.e*., those generated from monocyte's that have been cultured in GM-CSF and IL-4) are typically negative for the monocyte marker, CD14, and positive for the DC marker, CD1 a.

In contrast, the Transwell^{®}-derived DCs had a marker profile that included the expression of CD14 and a lack of CD1a. These opposing results might be explained simply by differences in culture conditions specific to each method. For example, the lack of CD1a on Transwell^{®}-derived APCs is not unexpected as it has previously been demonstrated that DCs derived in culture media containing human serum lack expression of this particular surface protein. We anticipate that APCs derived from Transwell^{®}s containing fetal bovine serum would express CD1a. If compared solely against cytokine-derived DC, the retention of CD14 on Transwell^{®}-derived APCs might suggest that these cells have not fully differentiated into DC. However, these results are consistent with other reports suggesting that monocytes triggered to differentiate into D.C via contact with endothelial cells do not lose CD14 expression (Randolph et al. (1998) Science 282, 480-483; Li et al. (2003) J Immunol. 171, 669-677). In fact, Li *et al.* demonstrated that endothelial cells may actively promote the expression of CD14 on these cells, as monocytes cultured on plate-bound P-selectin (an endothelial cell ligand), in addition to IL-4 and GM-CSF, gave rise to DCs that retained CD14. The retention of CD14 did not inhibit the APC function of these cells. Furthermore, CD14⁺ DC populations have been identified *in vivo.*

The flexibility of the system of the present invention makes it well suited for the study of different DC populations, such as those found in various tissue niches *in vivo.* While in the examples described here, HUVECs were used to drive the differentiation of monocytes into DCs, in other embodiments of the invention, other endothelial cell populations can be used within the Transwell^{®} system to preferentially drive the differentiation of monocytes into other tissue-specific DC subpopulations. For example, a previous report showed that intestinal epithelial cells cultured in a Transwell^{®} bucket gave rise to a unique DC population that lacked costimulatory and MHC class II molecules and were poor stimulators of T cell responses. This *in vitro* population resembled tolerogenic DCs found in the intestinal mucosa *in vivo.*

Monocytes that migrated through Transwell^{®}s containing brain endothelial cells had lower functionality than non-migrated monocytes (Seguin et al. (2003) J Neuroimmunol 135, 96-106), a result that contrasted with our findings with Transwell^{®}s containing HUVECs.

The modular design of the Transwell^{®} device allows for multiple embodiments that permit a greater dissection of DC development/differentiation pathways. For example, transmigrated APCs harvested from a Transwell^{®} can be passed through a second Transwell^{®e} chamber containing a monolayer of lymphatic cells, a process that more closely recapitulate the migration of matured/activated tissue-resident DCs through the lymphatics *in vivo.*

Additional cell types that might be involved in promoting the differentiation or function of DC can also be introduced into the system. For example, fibroblasts contained in the lower chamber of the Transwell^{®} device can serve as a source of inflammatory signals and act as an antigen depot during the application of certain adjuvants or pathogens. Other support cells, such as stromal cells, can also be contained in the lower chamber of the device of the present invention.

The specification describes a novel and convenient approach for generating large numbers of highly purified human DCs from blood monocytes. Using, for example, a flexible and well-characterized Transwell^{®} device as a support structure for the culture of endothelial cells and transmigration of monocytes through this confluent monolayer, a population of non-adherent APCs was generated that resemble other *in vitro* DC populations in phenotype and function. A transformed endothelial cell line may be promote the development of DCs. The specification describes methods which provide a simple means of generating human DCs in a manner that more closely mimics their development *in vivo.*

The present invention involves the use of a membrane device, for example, a commercially available Transwell^{®}, as a means of developing DCs to participate in an immune response. A non-immunogenic and biologically inert membrane with pores of a size that permit cell transmigration provides support for the growth of endothelial cells (*e.g*., human umbilical vascular endothelial cells (HUVEC) or other mammalian endothelial cells or cell lines), enabling the selective passage of monocytes through the membrane and concomitantly regulating and promoting their differentiation into DCs.

The membrane can be housed in an upper chamber suspended over and separable from a lower tissue culture well (chamber). An embodiment of the invention is shown in figure 1. In an embodiment, endothelial cells can be cultured to confluency on the porous membrane and then PBMC can be applied to the upper chamber (Fig. 1, step 1). About two days after leukocyte seeding, the upper chamber is removed and antigen, in the presence or absence of additional stimuli, is added to the lower chamber (Fig. 1, step 2). The DCs acquire the antigen and then can be used, for example, in T cell stimulation experiments or other APC functional assays.

In an embodiment of the present invention, using a porous membrane bearing an endothelial cell layer that is close to, or has achieved, confluent or even multilayer growth is a convenient method for developing dendritic cells for *in vitro* experimentation and *in vivo* therapeutics.

The membrane supports endothelial cell growth and can provide a barrier that selects for or enriches monocytes from peripheral blood cells. If, for example, peripheral blood cells are added to an upper chamber that has an endothelial cell-layered membrane as its bottom, such as in a Transwell^{®}, monocytes preferentially migrate through the cell-layered membrane and differentiate into DCs by virtue of their interaction with the endothelial cells (Qu et al. (2003) J lmmunol 170:1010-1018; Randolph et al. (1998) Science 282:480-483).

The transmigrated cells enter a lower chamber that is separate and free from the upper chamber, such that the possibility of "reverse transmigration" observed in the collagen substrate-endothelium model (Randolph et al. (1998) Science 282:480-483) is significantly reduced. Thus, antigen can be easily added to this separate compartment. for processing by the immature DCs. Additional agents, such as adjuvants, proinflammatory agents, vaccines, cosmetics, drugs, biologics, immunotherapy candidates, or chemical compounds, can also be added to the lower chamber to assess their effects, independently or together with antigen, on the activation and maturation of the transmigrated cells.

The modular design of the membrane device allows for multiple cell layers and different matrix materials, and other compounds such as cytokines or stimulants to be introduced into the system. The layers can be discrete and separable, thereby allowing the cells to undergo sequential processes without interference from the products or reactants of a previous event. For instance, monocytes that transverse an endothelial layer in the upper chamber can interact with an ECM (extracellular matrix) material in the lower chamber of the Transwell^{®} device (Fig. 2A). The ECM material used in any of the embodiments of the invention preferably comprises a material selected from the group consisting of gelatin, collagen, synthetic ECM materials, PLGA, PGA, natural ECM materials, chitosan, protosan, and mixtures thereof. Transmigrated DC that have processed antigen can also be passed through a second chamber with a membrane bearing a layer of lymphatic or other endothelial cell types on its top (Fig. 2B) or bottom (Fig. 2C). Alternatively, cells can be cultured on both the upper and lower sides of the membrane, such that monocytes pass through two cell monolayers before acquiring antigen or agents (as defined above) (Fig. 2D). ECM material could also be incorporated into this design (Fig. 2E) and also can be present with the endothelial cells being cultured on the membrane. In a dual-membrane device, monocytes will migrate through one cell layer, acquire antigen or agents (as defined above), and then migrate through a second cell population (Fig. 2F). The separable membranes with independent chambers allows for the easy addition of reactants and flexibility in the timing of events. In each of these example designs, the migration of monocytes through lymphatic endothelial cells can further promote their differentiation towards DC, similar to the maturation that occurs when the cells migrate into lymphatic vessels under physiologic conditions.

Additional cell types that might be necessary to promote the differentiation or function of DC could also be introduced into the system. For example, fibroblasts could serve as a source of inflammatory signals and act as an antigen depot during the application of certain adjuvants or pathogens. Other support cells, such as stromal cells, can also be contained in the system. Thus, these cells could be added to the lower chamber of the one-membrane device (Figs. 2G and 2H) or between the layers in a dual-membrane device (Figs. 2I and 2J). In this latter example, ECM can also be added between the membrane layers (Fig. 2J).

The process described here is scalable and automatable because Transwell^{®}s are commercially available in 12-, 24-, and 96-well plate and larger scale formats, and robotic liquid handling systems are available that can automate the transport of cells, liquids, chemical agents, or other materials between wells, and the removal of the upper Transwell^{®} chamber for access to the lower chamber.

Many sources of endothelial cells are suitable for use in the Transwell^{®} device. Primary endothelial cells are available from medical institutions and can be purchased commercially (*e.g*., Cambrex (East Rutherford, NJ) and VEC Technologies (Rensselaer, NY)). Although freshly thawed cells were used in the experiments described here, expanded primary cells can be used with similar results. Secondary (immortal) endothelial cells are convenient because of their longevity and rapid growth rates. For example, experiments suggest that EA.hy926, a long-term HUVEC line (Edgell et al. (1983) Proc Natl Acad Sci USA 80:3734-3737), and primary endothelial cells trigger transmigrated monocytes to undergo a similar differentiation program.

It has been shown that direct contact between monocytes and endothelial cells is required to promote their differentiation towards DCs (Qu et al. (2003) J Immunol 170:1010-1018), suggesting that surface-bound, but not soluble, proteins expressed by endothelial cells trigger monocyte differentiation. Thus, it is likely that cell membranes isolated from cultured endothelial cells, when tethered to the polycarbonate (PC) membrane, may be sufficient to promote monocyte differentiation. In such an embodiment, endothelial membranes could be stored long-term, either separately or already integrated into the Transwell^{®} device, eliminating the need for live endothelial cells.

Our findings on the use of endothelial cell-layered porous membranes or supports for the purpose of differentiating monocytes into DCs are contrary to some data in the literature. Specifically, Seguin *et al.* (2003) observed that monocytes that transmigrated through a brain endothelial cell layer on a porous membrane had no altered morphology and were equal to non-migrated monocytes in APC function, as assessed by their ability to stimulate allogeneic T cells (*J Neuroimminol* 135 *:*96- 106). In contrast, our data suggest that transmigrated monocytes differ phenotypically and functionally from non-transmigrated cells. The data presented in our examples confirm the use of a membrane-endothelial cell device for promoting the differentiation of monocytes towards a DC phenotype.

### EXAMPLES

### Example 1.

*HUVECs.* Primary HUVECs were obtained, for example, at passage #2 from VEC Technologies (Rensselaer, NY). Frozen stocks of primary endothelial cells were thawed and applied directly to 12-well Transwell^{®} devices (Coming, Corning, NY) at a density of ~9 × 10⁵ cells/cm² in MCBD-131 complete media (VEC Technologies). ~85% of the media was exchanged every other day and HUVECs were typically cultured on Transwell^{®} membranes for ~7 d before being used in monocyte migration assays. Although Transwell^{®}s with ~5 µm polycarbonate membranes were used for these assays, other membranes of various inert materials and/or pore sizes are also suitable.

### Example 2.

*HUVEC confluency.* The formation of tight-gap junctions in HUVEC monolayers was visualized by fluorescence microscopy. The staining process involved fixing the cells with 3.2% paraformaldehyde (32% stock from Electron Microscopy Science, Hatfield, PA) for ~10 min and permeabilizing them with methanol at ~20°C for ~5 min. The cells were then labeled with a 1:10 dilution of an antibody against human CD31 (M89D3; BD Pharmingen) for ~1 h at RT in a humidified chamber, followed by 1 mg/mL DAPI (Sigma) for ~5 min to label the nuclei. Next, the cells were fixed again with 3.2% paraformaldehyde for ~10 min at RT and then covered with GelMount (Biomedia, Foster City, CA). Extensive washes with phosphate-buffered saline (PBS) were included between steps. The labeled cells were examined using an Olympus IX81 fluorescence microscope. The permeability of the endothelial cell monolayer was measured by a standard diffusion assay. HUVECs were cultured on membranes as described above, except that the cells were switched into assay media (Iscove's modified Dulbecco's medium (IMDM; Mediatech, Inc., Herndon, VA), containing 5% heat-inactivated (56°C, 30 min.) autologous or human AB serum, 2 mM L-glutamine, 100 U/ml penicillin, and 0.1mg/ml streptomycin) 24 h prior to, and diffusion media (1MDM) supplemented with 1 % BSA) 1 h before the start of the diffusion assay. FITC-conjugated dextran (70 kDa; Sigma) diluted to 1 mg/mL in diffusion media was added to the upper well and four 100 µL aliquots were taken from the lower well at 30 min intervals. To avoid changes in hydrostatic pressure, an equal volume of fresh diffusion media was added to the lower chamber after the samples were removed. The fluorescence of the media samples were measured with a Bio-Tek (Winooski, VT) Synergy HT spectrophotometer, using a 480/520 nm filter set. A standard curve, established by measuring the fluorescence of known amounts of FITC-dextran, was used to calculate the concentration of dextran that permeated through the HUVEC monolayer.

### Example 3.

Transendothelial electrical resistance (TEER) was used as a second method to examine the integrity of the HUVEC monolayer. Endothelial cells were cultured on Transwell^{®} membranes in MCBD-131 complete media, switched into assay media for 24 h, and then TEER was calculated with a Voltohmeter (EVOM-ENDOHM-6, World Precision Instruments, Sarasota, FL) using a resistance chamber compatible with the Transwell^{®} inserts. The voltohmeter was calibrated each day, per the manufacturer's instructions, and 3 individual readings were taken for each well. The TEER readings of the HUVEC monolayers on Transwell^{®} membranes were normalized against values collected from Transwell^{®} inserts alone (in the absence of endothelial cells).

### Example 4.

*Human PBMC preparation**.*** Enriched leukocytes were obtained from the Central Florida Blood Bank (Orlando, FL). All of the donors were in good health and all blood products were negative for blood-borne pathogens, as detected by standard assays. PBMCs were enriched by density centrifugation. Briefly, ~45-50 mL leukocytes were resuspended in citrate buffer (PBS containing 0.1% BSA and 0.6% Na citrate) to a final volume of ~140 mL. Diluted blood (~35 mL) was layered onto ~15 mL Ficoll-Paque PLUS (Amersham Biosciences, Piscataway, NJ) in a 50 mL conical tube and centrifuged (400 g, 25 min, room temperature). The buffy coats were removed, washed twice with citrate buffer, recentrifuged (400g, 10 min, 4°C), and resuspended in assay media. The PBMC were kept at 4°C for up to 24 h prior to being used in assays or were frozen and stored in liquid nitrogen for extended storage.

### Example 5.

*Monacyte transmigration assays.* PBMC were applied to confluent endothelial cells that had been transferred into assay media ~24 h earlier. ~10 × 10⁶ total PBMC were applied to each 12-well Transwell^{®} and incubated for ~1.5 h. The upper chambers were washed twice with assay media to remove non-adherent and loosely bound cells, and the Transwell^{®} plates were incubated for an additional ~48 h to allow for leukocyte transmigration and differentiation. The upper chambers were then removed and the cells in the lower chamber were harvested for phenotypic or functional analyses.

### Example 6.

*DC phenotyping* PE-, APC-, or PerCP-Cy5.5-conjugated monoclonal antibodies specific for human CD1a (HI149), CD14 (M5E2) CD16 (3G8), CD40 (5C3), CD80 (L307.4), CD86 (2331), CD83 (HB15e), and HLA-DR (L243) were purchased from BD Pharmingen and diluted as suggested by the manufacturer. Isotype controls included MlgG2a (G155-178) and MIgG1 (MOPC-21), which were also purchased from BD Pharmingen. ~1-2×10⁵ transmigrated cells from HUVEC-negative and - positive Transwell^{®}s were collected at various times following PBMC seeding and labeled with specific antibody for ~45 min at 4°C, washed extensively, and fixed with 2% paraformaldehyde. The buffer used for cell labeling was PBS with 2% BSA and 0.05% sodium azide. Samples were acquired on a FACSArray (BD Pharmingen) and FlowJo software (Treestar, Ashland, OR) was used for analysis.

### Example 7

*T cell stimulation assay.* About two days after PBMC were applied to the HUVEC monolayer, the transmigrated cells were pulsed with ~20 µg/mL *Candida albicans* protein antigen extract (Greer Laboratories, Lenoir, NC). ~48 h later, transmigrated cells were collected, washed, and combined with syngeneic T cells. Cytokine-derived DCs were prepared using standard procedures. Briefly, monocytes were purified from total PBMC using anti-CD14 antibody-conjugated magnetic beads (Miltenyi Biotec) and then cultured for ~7 d at ~1 ×10⁶/ml in assay media containing ~100 ng/mL GM-CSF (R & D Systems, Minneapolis, MN) and ~25 ng/mL IL-4 (Endogen, Rockford, IL). The cells were pulsed with ~20 µg/mL *Candida albicans* on day 5 of culture.

Frozen stocks of syngeneic PBMC were used as a source of lymphocytes. Total T cells were purified by negative selection, using magnetic beads and the autoMACS system (Miltenyi Biotec (Auburn, CA)). Purified T cells were washed with PBS, labeled with 5 µM CFDA-SE (CFSE; Invitrogen, Carlsbad, CA), and then washed two times with assay media, to quench the labeling reaction. The cells were plated at ~2-3 × 10⁵/well in 96-well flat-bottom tissue culture plates (Corning, Inc., Corning, NY) and DCs were added at the indicated ratios. Each well contained a final volume of ~200 µL.

The leukocyte cocultures were incubated for ~7 d at 37°C and 5% CO₂ and then the activated T cells were tested for intracellular cytokine production (*i.e*., antigen specificity) using standard procedures. Target APCs (cytokine-derived DCs) were prepared as described above. On day 5, a fraction of the cells was pulsed with 20 µg/mL *Candida albicans* and then on day 6, these cells were further activated by adding 25 ng/mL TNFα (Endogen). On day 7, target DCs were cultured with activated T cells for -8 h at a ~1:10 ratio in the presence of 1 µg/mL brefeldin A (Sigma, St. Louis, MO). The cells were surface-labeled with an antibody specific for CD3ε (SK7; BD Pharmingen), and then intracellularly labeled with an antibody specific for human IL-2 (Endogen) using Cytofix/Cytoperm and perm/wash reagents from BD Pharmingen.

### Example 8.

It has been suggested that a confluent HUVEC layer is required to facilitate the differentiation of transmigrated monocytes into dendritic cells (Qu et al. (2003) J Immunol 170:1010-1018*;* Randolph et al. (1998) Science 282:480-483). As such, endothelial cells grown on Transwell^{®} membranes were examined at several time points post-seeding for the formation of tight-gap junctions, indicative of quiescent endothelial cells (Dye et al. (2001) Microvasc Res 62:94-113). Although the cells were seeded at a density sufficient to form a confluent layer within ~1-2 days, the formation of tight-gap junctions, as demonstrated by surface CD31 (PECAM-1) fluorescence (Dusserre et al. (2004) Arterioscler Thromb Vasc Biol 24:1796-1802), was not evident until day ~3-4 (Fig. 3A illustrates cells at 7 days post-seeding). DAPI was used to counterstain the cell nuclei (Fig. 3A, right panel). It is also well established that the formation of tight-gap junctions in endothelial cells is associated with increased transendothelial resistance (TEER) and decreased diffusion across the monolayer. Thus, the results of Fig. 3B demonstrating that TEER increased dramatically between about days 3 and 4 post-seeding, and Fig. 3C, which highlights a loss of FITC-dextran diffusion through the HUVEC between days 2 and 7 following PBMC application, further support the conclusion that endothelial cells could be cultured to confluency/quiescence on polycarbonate- Transwell^{®} membranes. In subsequent experiments, the Transwell^{®}s were used at 7 days after HUVEC seeding.

### Example 9.

The effect of endothelial cells on monocyte transmigration and differentiation was assessed using published protocols (Qu et al. (2003) J Immunol 170:1010-1018; Randolph et al. (1998) Science 282:480-483) that were modified to fit the Transwell^{®} system. An important observation from previous studies is that monocytes can be significantly enriched from total blood leukocytes that are applied to quiescent endothelial cells on a collagen matrix because they cross the HUVEC monolayer more quickly and in greater numbers than other cell types (Randolph et al. (1998) Science 282:480-483). Similarly, when PBMCs were applied to a confluent endothelial monolayer on a Transwell^{®}-PC membrane, nearly all of the transmigrated cells at ~2 d post-seeding were uniform in size and had processes/veils extending from the cell body that are characteristic of DC (Fig. 4A, right panel). In contrast, the absence of an endothelial cell monolayer allowed for the transmigration of a more heterogeneous population, including cells that morphologically resembled erythrocytes and lymphocytes, through the Transwell^{®} membrane (indicated by arrows in Fig. 4A, left panel).

### Example 10.

To determine whether HUVECs affect the differentiation state of transmigrating monocytes, cells that passed through a Transwell^{®} membrane in the presence or absence of an endothelial cell layer were labeled with antibodies specific for surface proteins characteristic of DCs. Because it was possible that the simple process of migrating through a porous membrane might trigger an altered phenotype in monocytes, non-migrated cells were included for comparison. To this end, CD14-positive monocytes were cultured for ∼2, d in assay media without any exogenous cytokines and then profiled by flow cytometry (Fig. 4B). The results of this experiment are consistent with previous reports showing that circulating monocytes are positive for some APC markers, such as HLA-DR and CD86, but are negative for others, including CD40 and CD80 (Elkord et al. (2005) Immunology 114:204-212; Salek-Ardakani et al. (2004) J Immunol 173:321-331). The median fluorescence intensity (MFI) of surface proteins on non-migrated monocytes shown in Fig. 4B was used to establish a baseline profile against which migrated monocytes were compared (Fig. 4C).

### Example 11.

Monocytes differentiated into DCs in the presence of exogenous cytokines typically lose the membrane CD14 and acquire the immature DC marker, CD1a. That transmigrated DCs had a different profile was not surprising, however, because CD1 a typically remains low and CD14 is not lost on monocyte-derived DCs that are cultured in human serum (Piemonti et al. (2000) Cancer Immunol Immunother 49:544-550): The low expression of CD83, a marker of mature DCs, on transmigrated cells suggests that they are in a largely immature state (Fig. 4C). The other molecules included in this analysis are important for antigen acquisition and T cell costimulation. Specifically, the low affinity IgG receptor, FcγRIII (CD 16), which was upregulated on monocytes that migrated through the HUVEC layer, plays an important role in the uptake of antibody-coated proteins. CD86 and CD80 provide important stimulatory signals for naive T cell activation and proliferation. Although CD86 was already expressed at a high level on purified monocytes (Fig. 4B), CD80 was negative on non-migrated monocytes and only increased after monocytes migrated through an endothelial monolayer (Figs. 4B and 4C, respectively). Similar results were obtained for CD40, a marker that provides maturation signals to the DC itself.

### Example 12

While surface markers expressed by DCs can be useful in distinguishing them from other cell types, the defining characteristic of APCs is their ability to trigger T cell responses. The functionality of Transwell^{®}-derived DCs was gauged against cytokine-derived DCs from the same donor in a syngeneic T cell stimulation assay. Both cell types efficiently triggered T cell proliferation (CFSE dilution) and elicited a similar frequency of effector cells that were capable of secreting IL-2 following short-term antigen stimulation (Fig. 5). This response was likely antigen-specific as the only T' cells capable of secreting IL-2 at levels above background were those that encountered *Candida albicans* during both the 7-day stimulation and 8 h ICCS assay (Fig. 5).

### Example 13.

To ensure that the process of migrating through a membrane alone was not sufficient to trigger potent APC activity in monocytes, transmigrated cells that had passed through a membrane in the absence and presence of HUVECs were tested for their capacity to trigger T cell responses. The results demonstrated that the interaction of monocytes with endothelial cells was necessary to promote their complete differentiation, because cells that passed through the membrane alone had no antigen-specific T cell stimulatory activity above background. The disparity in the frequency of T cells that respond to Transwell^{®}-derived DCs in Figs. 5A and 5B is likely due to the immune history of the donors that were used in these independent experiments.

### Example 14.

In an embodiment of the present invention, antigenic molecules introduced into an artificial immune system (AIS) are acquired by dendritic cells (DCs) at the vaccination site (VS). The DCs are then transferred to the lymphoid tissue equivalent (LTE), where they present the antigen to T cells, activating their immune function. Activated helper T cells co-stimulate B cells to induce antibody production, while activated cytotoxic T cells lyse antigen-bearing cells. Solublized antigen can also be introduced to the LTE to directly activate B cells.

Integration experiments were performed using DCs from a Transwell^{®}-based vaccination site (VS) model that were placed into the microcarrier lymphoid tissue equivalent (LTE). These APCs are able to present antigen to T cells and show antigen-specific T cell responses (proliferation). The results are compared to those observed in 2D culture dishes.

### Example 15.

*Vaccination Site.* In embodiments of the present invention, various configurations have been used with collagen, a porous polycarbonate membrane, incorporation of antigen into a membrane-based model, and the ability to increase the complexity of a membrane based VS model in a manufacturable manner (*e.g*., the addition of stromal cells, the addition of an epithelium, *etc.*)*.* The porous polycarbonate (PC) membrane can act as a support layer for the extracellular matrix (ECM), such as collagen (see examples in Figure, 6).

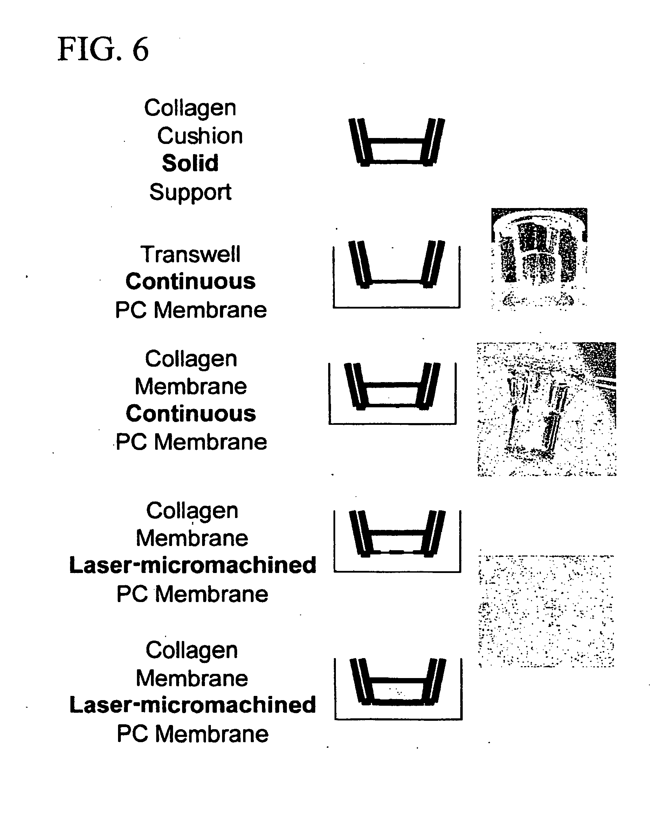

The effects of these variables on the phenotype and the numbers of transmigrated antigen-specific DCs has been examined. Experimental variables examined include the configurations shown in Figure 6.

In embodiments of the present invention, we examined a collagen cushion in a standard 96-well plate model, a simple Transwell^{®}-based model, a model with a collagen matrix integrated with a filter membrane, a model with the polycarbonate membranes laser-micromachined to increase porosity and potentially cell flux, and a model in which two endothelial layers were created, one on the top and one on the bottom of the VS membrane construct to examine the influence of one endothelial layer versus two endothelial layers on cell migration pathways, cell migration numbers, DC phenotype, and DC function.

### Example 16_{.}

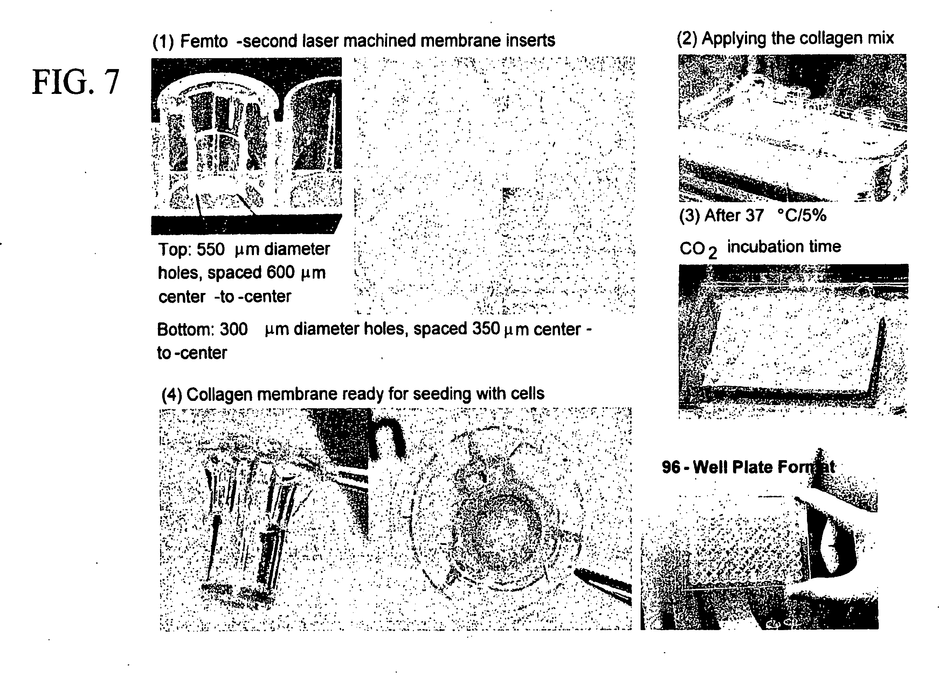

In an embodiment of the present invention, a collagen membrane was cast in a well-based format. We have developed a method to cast collagen in a membrane format in a simple well-based system. As examples, we have examined three support structures with the collagen membrane: a continuous polycarbonate (PC) membrane (~8µm pore diameter), a laser-micromachined PC membrane (a range of pore diamaters available [∼100-550 µm]), and a nylon mesh (a range of mesh size available [~100-500 µm]). Figure 7 shows examples of the laser-micromachined PC membranes and steps taken to create the collagen membrane in either a 96-well format or a simple single-well format.

### Example 17.

We examined whether the collagen and/or PC membrane impeded cell migration. We examined cell migration in a model comprising a collagen membrane on a continuous PC membrane support (third model in Figure 6). A confluent HUVEC monolayer was grown on top of the collagen membrane and PBMCs were added for monocyte selection and migration through the HWEC layer.

After ∼1.5 h, non-migratory cells were washed off and the migratory cells were left in the construct for ∼48 h to allow for reverse-transmigration back through the HUVEC layer, transmigration through the collagen and PC membrane, or retention within the collagen membrane. We found that even though the cell migration numbers were lower for the cells that transmigrate through the collagen and the PC membrane compared to those that reverse-transmigrate back up through the endothelial cell layer on top of the collagen, neither the collagen nor the PC membrane impeded cell migration.

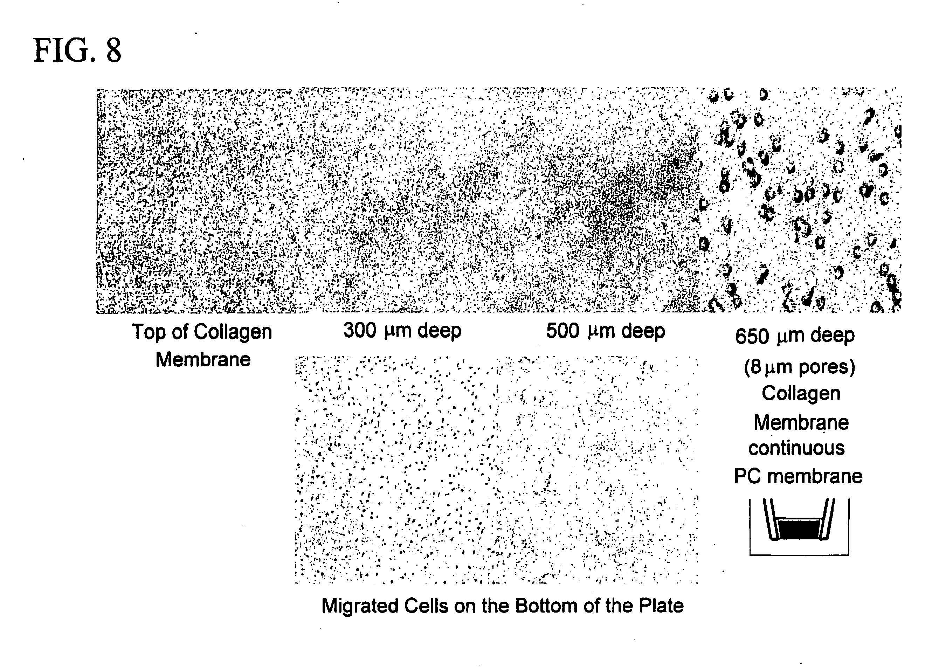

Figure 8 shows the appearance of migratory cells throughout the collagen membrane, as well as cells on the bottom of the plate that migrated completely through the construct. Cell migration through the constructs also depended on other factors such as collagen density, the thickness of the collagen membrane, and adding a second HUVEC layer to the bottom of the construct.

### Example 18.

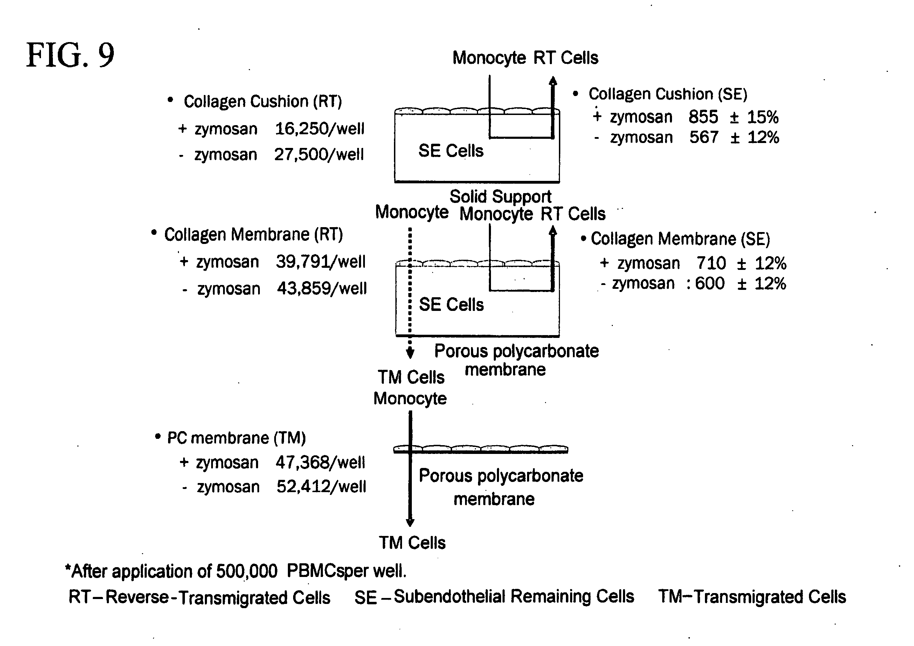

In various vaccination site models, we examined the effects of some of the design variables on the phenotype and the numbers of transmigrated cells. We compared the phenotype and cell numbers of transmigrated cells from the model consisting of collagen on a continuous PC membrane (the third model shown in Figure 6) with the collagen cushion model (the first model shown in Figure 6), and the Transwetl^{®}-based model (the second model shown in Figure 6). Figure 9 shows cell numbers from each model.

### Example 19.

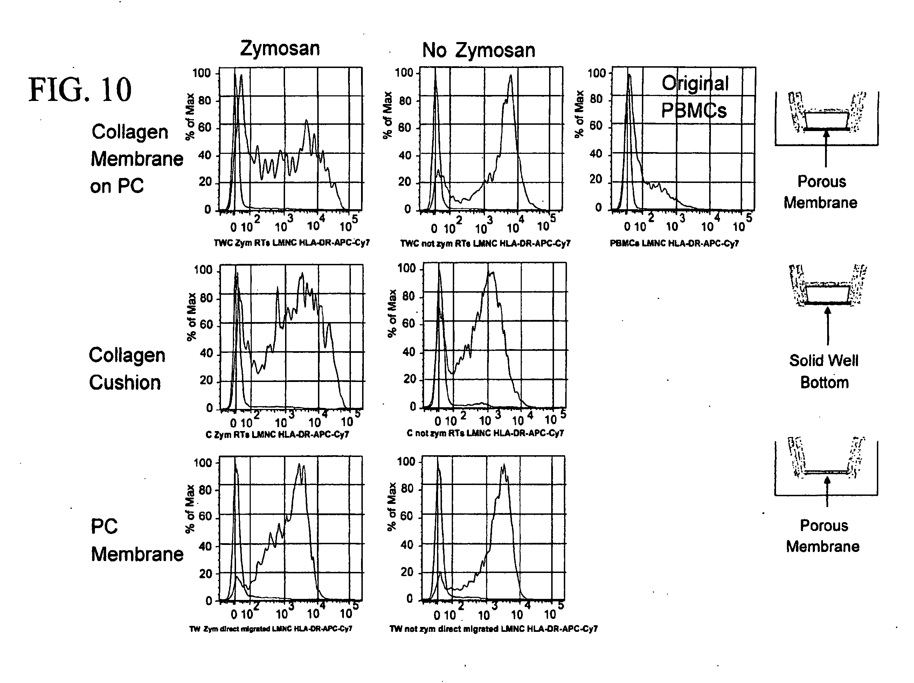

We also compared the DC phenotype of the cells that migrated from each of the three models. Figures 10, 11, and 12 show the levels of expression of HLA-DR, CD86, and CCR7 of the migrated cells each model, respectively. As the DCs produced from the VS are of an immature phenotype, zymosan (known to mature DCs) was added as a test sample for each model to compare the mature DC phenotype from each of the three models.

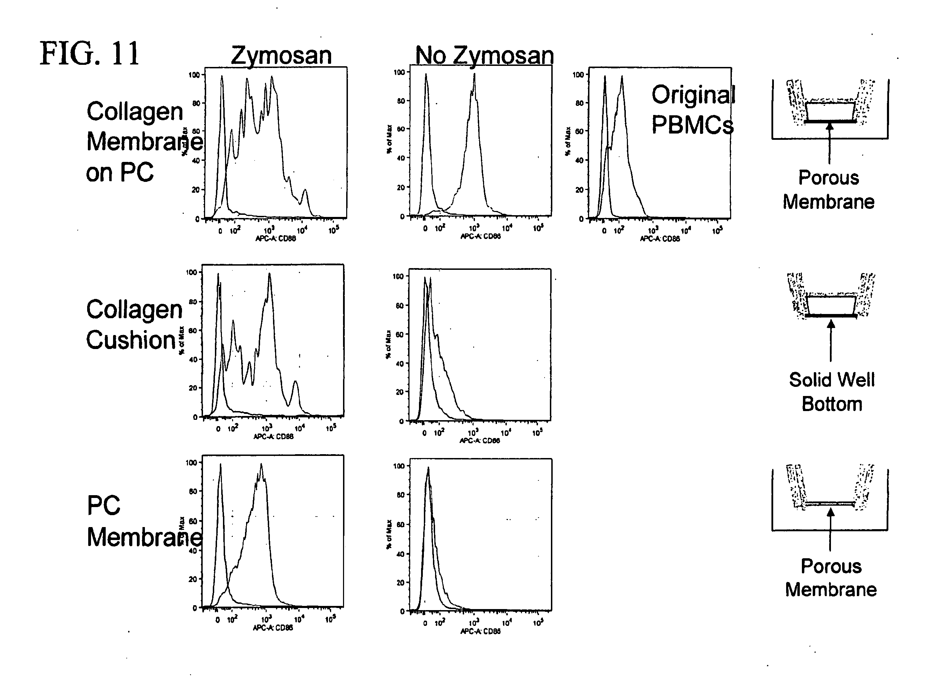

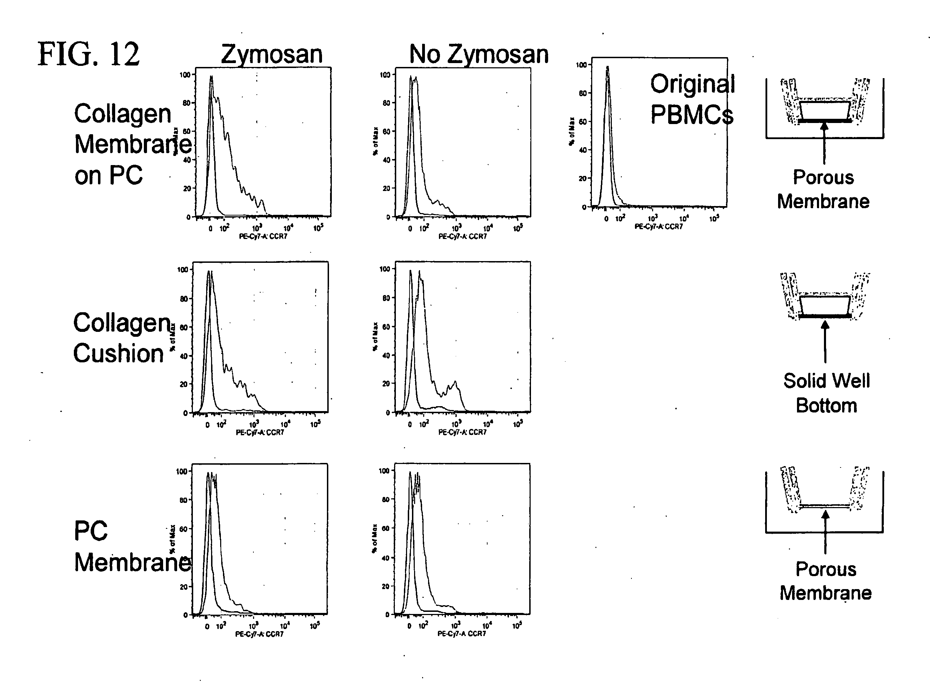

As shown in Figure 10, the levels of HLA-DR expression for the mature migrated cells (exposed to zymosan) from the collagen on PC membrane model was very similar to that of the collagen cushion model, and both were higher than that seen for the Transwell^{®} model. The same was also seen for the levels of CD86 and CCR7, shown in Figures 11 and 12, respectively. The phenotype analysis showed that the cells migrating from the model of collagen on a PC membrane have a similar phenotype to those migrating from the collagen cushion model and we expect them to function similarly.

### Example 20.

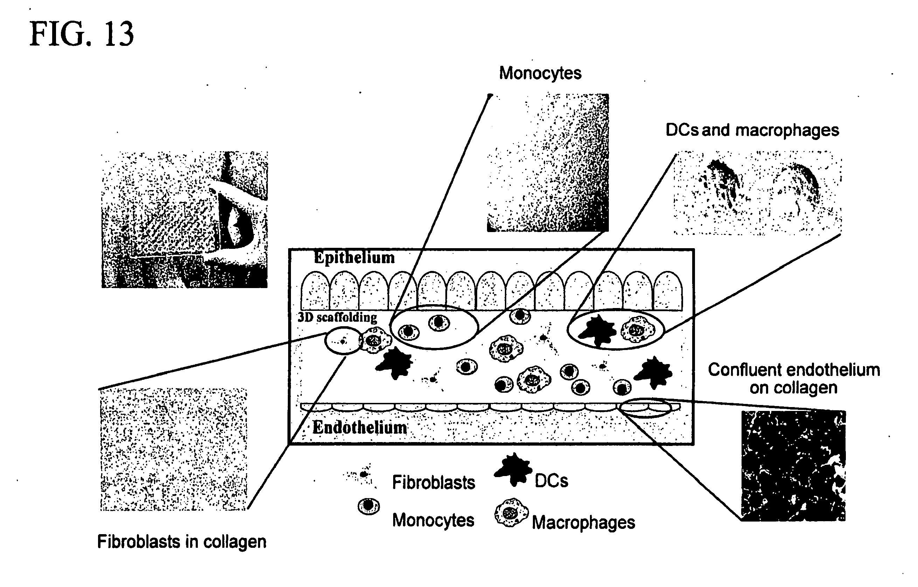

*The ability to build-in complexity.* In Figure 13, we show how complexity can be added. As an example, we can form a confluent endothelium over the collagen membrane, we have been able to observe monocyte transendothelial migration into the collagen membrane, we have been able to observe monocyte differentiation into DCs and resident macrophages, we have been able to introduce fibroblasts into the collagen, and we have been able to show that these embodiments can be manufactured in, for example, a 96-well format.

### Example 21.

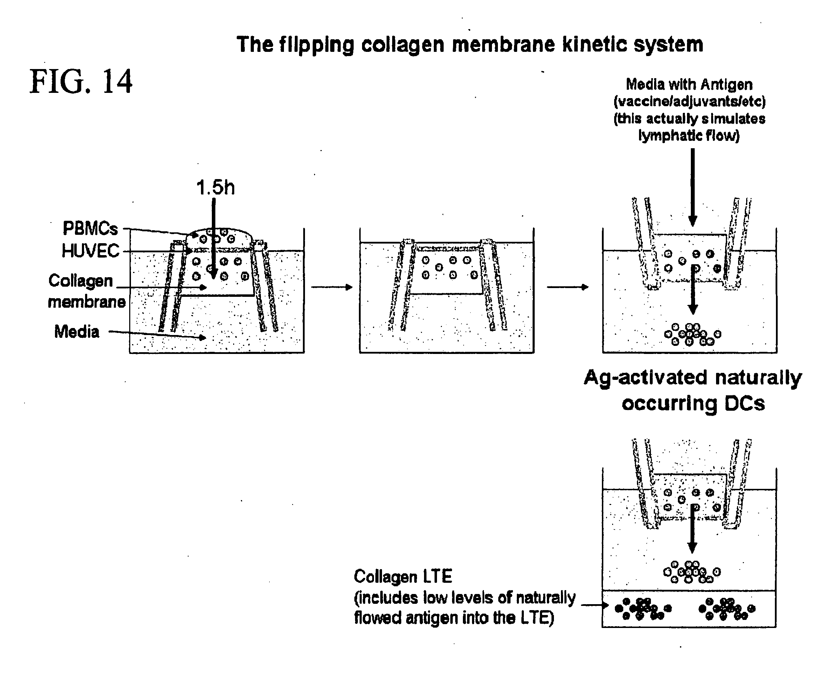

*Antigen introduction into the VS.* Figure 14 shows an example of how antigen can be added to a membrane-based AIS integrated into a well-based format. This figure shows a means of introducing antigen into a collagen membrane with a confluent HUVEC layer present. Once the HUVECs are seeded and grown to confluency, PBMCs are applied to the HUVEC face and allowed to extravasate through the endothelium. After ~1.5 h (typical protocol), non-migratory cells were washed off the endothelium surface and the bucket/well can then be inverted and placed into the LTE section of an AIS system. The antigen, the vaccine and/or adjuvants can then be introduced through the back side of the inverted VS construct. Maturing DCs can then migrate out of the VS and fall into the LTE below. Antigen uptake occurs while the monocyte-derived DCs are in the collagen membrane. An additional benefit of this approach is that solubilized antigen that is not engulfed by APCs, can also fall into the LTE where it can be processed directly by B cells.

### Example 22.

Primary HUVEC cultures were grown in MCDB-131 complete media, containing 10% fetal bovine serum, 10 ng/mL endothelial growth factor, 1 µg/mL hydrocortisone, 0.2 mg/mL ENDOGRO, 0.1 mg/mL heparin, and an antibiotic/antimycotic solution (all reagents from VEC technologies). The transformed endothelial cell line, EA.hy926 (Edgell et al. (1983) Proc Natl Acad Sci USA 80, 3734-3737), was a gift from Cora-Jean Edgell (University of North Carolina at Chapel Hill, Chapel Hill, NC). These cells were grown in M199 media (Invitrogen), containing 10% fetal bovine serum and passaged ~1:10 every -6-7 days.

All immune cell cultures and assays were performed in Iscove's modified Dulbecco's medium (IMDM; MediaTech), supplemented with 0.2 mM L-glutamine, 100 U/mL penicillin and 0.1 1mg/mL streptomycin (all from Sigma), and varying concentrations of heat-inactivated (56°C, 30 min) human plasma or fetal bovine serum (HyClone Laboratories).

### Example 23.

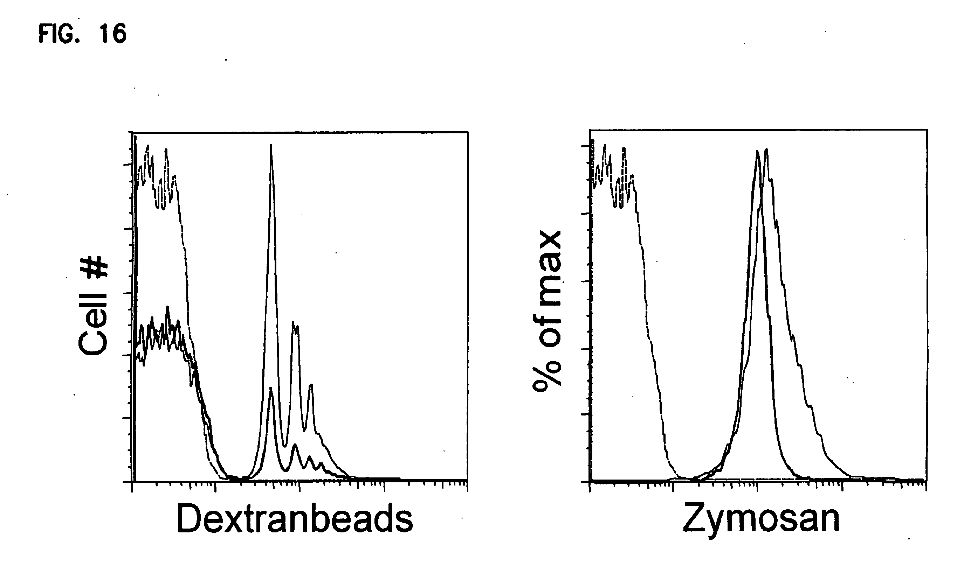

Transmigratory monocytes were collected ~2 d after PBMC application and incubated overnight with 1 µm-diameter orange fluorescent beads or AlexaFluor 488-labeled zymosan particles at a ratio of~3:1 to the cells (both reagents from Molecular Probes). Then, the cells were washed once in FACS buffer and analyzed by flow cytometry. In some cases, the APCs were treated with 20 µg/µL cytochalasin D for 2 h at 37°C prior to incubation with the beads or particles to block phagocytic activity (Fig. 16).

### Example 24.

Previous studies have shown that HUVEC grown to confluency on a collagen substrate create a highly restrictive barrier for the migration of most PBMC populations, except monocytes, through the endothelial monolayer (Randolph et al. (1998) Science 282, 480-483). Similarly, when PBMCs were applied to confluent HUVECs in the upper Transwell^{®} bucket, nearly all of the transmigrated cells were uniform in size and morphology (Fig. 4A, right panel). In contrast, the absence of a HUVEC monolayer permitted a more heterogeneous PBMC population, including erythrocytes and lymphocytes, to pass through the PC membrane into the lower Transwell^{®} chamber (Fig. 4A, left panel). (The use of non-adherent plates in this particular assay prevented any of the transmigrated monocytes from binding to the lower Transwell^{®} chamber.) When between ~1-5× 10⁶ PBMC were applied to the upper Transwell^{®}chamber, approximately 10% of the leukocytes transmigrated through the HUVECs. When the cultures were established in standard tissue culture-treated plastic dishes, about 50% of the transmigrated cells were low/non-adherent, while the other half exhibited strong adherence and morphologically resembled macrophages (data not shown).

### Example 25.

Previous studies suggest that monocytes which have made two passes through a confluent endothelial cell monolayer differentiate into APCs that resemble classical DCs in phenotype and function (Qu et al. (2003) J Immunol 170, 1010-1018; Randolph et al. (1998) Science 282,480-483). We sought to determine whether a single migration of monocytes through a confluent HUVEC layer, as occurs in the Transwell^{®} system, is sufficient to promote their differentiation towards iDCs. To this end, transmigrated APCs were collected from the lower Transwell^{®} chamber -48 h after PBMCs were applied to the upper chamber and examined for characteristic features of DCs. For many of these analyses, the role of endothelial cells in regulating the differentiation state of monocytes was examined by comparing cells that had migrated through PC membranes in the absence or presence of a HUVEC monolayer.

### Example 26.

Immune cells, and the various activation/maturation states of these populations, are often defined by their expression of a particular pattern of surface proteins. Therefore, the impact of HUVECs on monocyte differentiation was examined initially by comparing the phenotype of transmigrated monocytes that had contacted endothelial cells with those that had passed through an empty Transwell^{®} bucket. As it was possible that monocytes passing through a porous PC membrane in the absence of a HUVEC layer might also experience a change in their marker profile, non-migrated CD14⁺ cells that had been cultured for two days in assay media absent of exogenous factors were used to establish a baseline expression level for each marker of interest. In Fig. 4B, the median fluorescence intensity (MFI) of markers on the *non-migrated* monocytes was set at 100% and compared against the change in MFI of the same markers on monocytes that had *transmigrated* through the PC membrane 48 h earlier. The presence of a HUVEC monolayer caused a marked increase in expression of two molecules, CD40 and CD80, on the transmigrated APCs that provide critical costimulatory/activating signals to DCs and T cells, respectively. The low affinity IgG receptor, FcγRIII (CD16), which is important for the uptake of antibody-coated proteins, was upregulated on APCs that migrated through a HUVEC layer, though it was also elevated to a lesser extent on cells that passed through a PC membrane lacking an endothelial monolayer. The minimal increases in expression of CD86 and HLA-DR on transmigrated monocytes was not surprising since these proteins were already expressed at a high level on non-migrated monocytes (data not shown). Transwell^{®}-derived APC were unlike traditional cytokine-derived DC in their retention of the monocyte marker, CD14, and lack of the DC marker, CD1a (Fig. 4B). Flow cytometric data for monocytes that had transmigrated through a confluent endothelial monolayer, which was shown graphically in Fig. 4B, is shown in histogram form in Fig. 4C.

### Example 27.

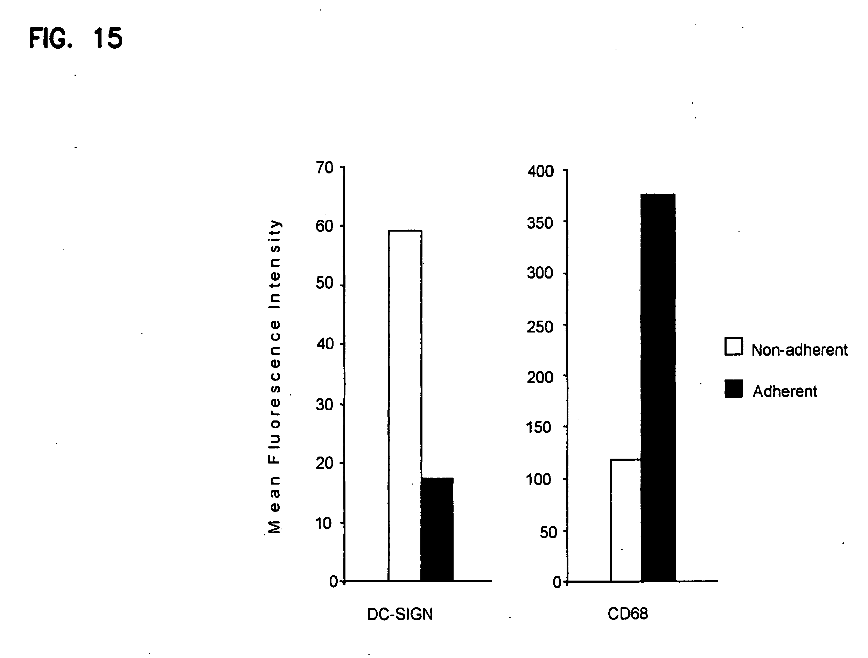

*In vivo* and *in vitro* data indicate that monocytes can differentiate into either macrophages or iDCs (Randolph et al. (1998) Science 282, 480-483). In keeping with these reports, it was evident, by morphology and adherence, that the transendothelial migrated cells comprised at least 2 distinct populations (data not shown). Phenotype analysis (Fig. 15) revealed several distinctions between the migrated adherent and non-adherent cells. The low-level expression of the DC marker, DC-SIGN, coupled with a high expression of CD68, on the adherent population suggests that these cells are indeed macrophages. The opposite phenotype of these markers on the non-adherent cells, specifically the elevated expression of DC-SIGN, further supports our contention that these transmigrated cells are differentiating towards DCs.

### Example 28.

The increased expression of costimulatory ligands on Transwell^{®}-derived cells suggested that a single transendothelial migration might be sufficient to trigger the differentiation of these cells into potent APCs. Additional experiments were performed to determine whether the changes in phenotype were also associated with increased functionality of the Transwell^{®}-derived cells. For instance, a defining characteristic of DCs is their ability to capture soluble and particulate material for MHC class I and II processing and presentation. The ability of APCs to acquire fluorescently labeled ~1 µm latex beads and zymosan (yeast) particles is indicative of strong phagocytic activity. As shown in Fig. 16, zymosan particles were captured by nearly all of the Transwell^{®}-derived APC, and about 30% of the cells acquired latex beads. While both materials are captured via mannose receptors, it is possible that the reduced accumulation of latex beads within the APCs could be related to the size of the beads, a it has been previously noted that small (~0.2 µm) beads are far more efficiently phagocytosed than larger beads. On the other hand, the increased efficiency of yeast particle uptake by the Trdnswell^{®}-derived cells could be mediated by other receptors, such as TLR2. The addition of cytochalasin D, an inhibitor of phagocytosis, triggered a partial reduction in the uptake of both materials by the Transwell^{®}-derived APCs, suggesting that the particles were ingested by an active mechanism.

### Example 29.

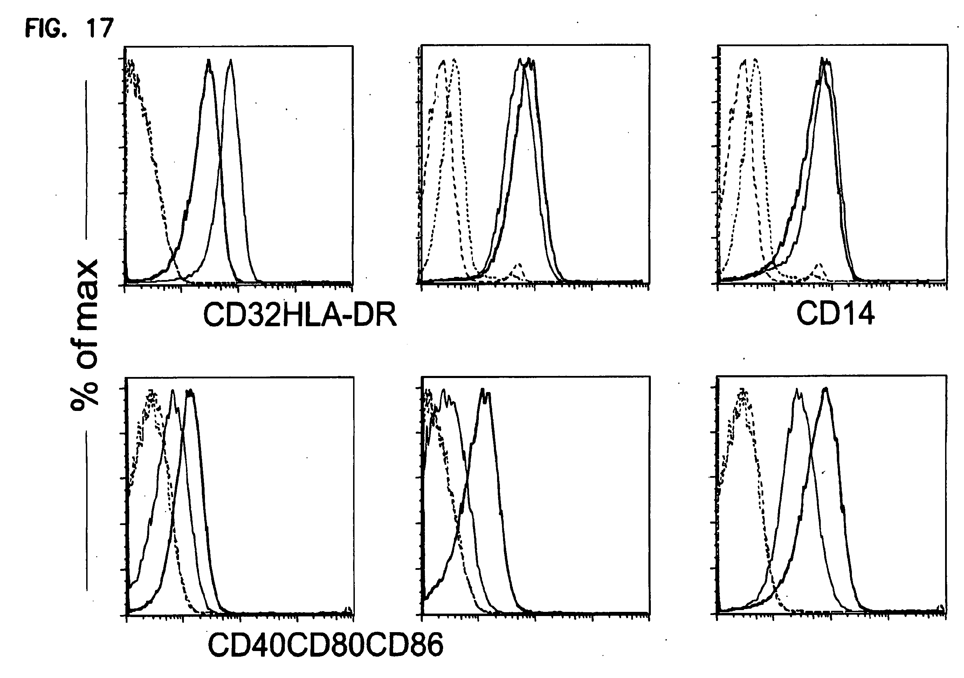

Another hallmark feature of DCs is their ability to undergo a maturation/activation program, which includes an altered expression of molecules associated with antigen presentation and T cell stimulation, following an encounter with various inflammatory stimuli. To assess the maturation potential of Transwell^{®}-derived APCs, migrated cells harvested from the lower chamber were stimulated for ~24 h with TNF-α and LPS and analyzed by flow cytometry for changes in their surface marker profile (Fig. 17). Markers associated with antigen uptake, such as the low affinity Fc receptor, CD32, decreased on activated DC, while others, such as CD40, CD80 and CD86, that serve important costimulatory functions for the induction of adaptive immunity, were elevated on the TNF-α/LPS-treated cells. The fact that MHC class II (HLA-DR) and CD14 were unaffected by the maturation stimuli further highlights the unique phenotype of Transwell^{®}-derived APCs, as cytokine-derived human matured DCs are typically triggered to upregulate MHC class II and further downregulate CD14 (data not shown).

### Example 30.

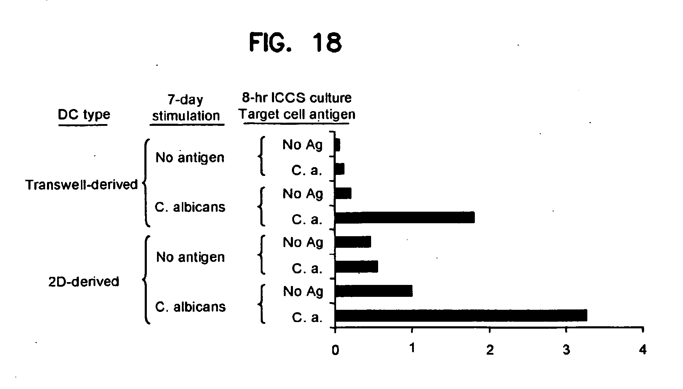

The important feature that distinguishes DCs from other APC populations is their ability to stimulate antigen-specific T cell responses. For this reason, transendothelial-migrated APCs from the Transwell^{®} device were evaluated for their ability to induce antigen-specific T cell responses, including lymphoproliferation and effector function. *Candida albicans (C. albicans),* a component of the natural microflora in humans, was chosen as an antigen source for these assays. Transwell^{®}-derived APC were pulsed with a whole protein antigen from *C*. *albicans,* matured with TNF-α, and then cultured for ~7 d with autologous T cells that had been labeled with the proliferation-sensitive dye, 5-(and-6-)-carboxyfluorescein diacetate, succinimidyl ester (CFDA-SE; CFSE). Thereafter, the T cells were evaluated for proliferation (CFSE dilution) and the production of cytokines following short-term TCR stimulation with target APCs that had been pulsed with specific antigen. The presence of *C*. *albicans-specific* CFSE^{low}IL-2⁺T cells, following a short-term antigen restimulation, provides strong evidence of the capacity of the Transwell^{®}-derived APCs to trigger the complete differentiation of antigen-specific T cells into fully competent effectors. Controls in this assay included DCs stimulators and targets that had not been pulsed with *C*. *albicans* antigens (Fig. 18). The quality of the Transwell^{®}-derived APCs as stimulators ofT cell responses was gauged against cytokine-derived DCs that were prepared from the same donor. The results of Fig. 18 demonstrate that Transwell^{®}-derived APC are nearly equal to classic DCs in their ability to trigger T cell responses, since both APC types elicited a similar frequency of *C*. *albicans-specific* effecter cells that were capable of secreting IL-2 following TCR ligation.

### Example 31.

Although, in our hands, non-migrated monocytes were unable to trigger specific T cell responses (data not shown), we considered the possibility that monocytes which had passed through a PC membrane in the absence of a HUVEC monolayer might have strong T cell stimulatory capacity. The results of Fig. 4C demonstrate, however, that the interaction of monocytes with endothelial cells is important to promote their complete differentiation into APCs, because cells that passed through a PC membrane alone were unable to trigger specific T-cell responses above background. The disparity in the frequency of T cells that responded to Transwell^{®}-derived DCs in Figs. 4B and 4C is likely due to differences in the immune histories of the two donors that were used in these experiments. The increased T cell stimulatory capacity of transendothelial-migrated monocytes could be related to the increased expression of costimulatory ligands, namely CD40 and CD86, on the Transwell^{®}-derived APCs (Fig. 4C), though further experimentation will be necessary to confirm this possibility.

### Example 32.

A constraint on the overall utility of the Transwell^{®}-based system described here is the use of primary HUVEC to drive the monocyte to DC differentiation. To overcome the use of these slow-growing cells, we repeated this series of experiments with a durable, fast-growing transformed endothelial cell line, EA.hy926, that was derived by fusing HUVEC with a human lung carcinoma cell line {Edgell, 1983 #24} (Edgell et al. (1983) Proc Natl Acad Sci USA 80, 3734-3737). In data not shown, the EA.hy926 cells grew to confluency on PC membranes and formed tight-gap junctions more quickly than the HUVECs. The transendothelial-migrated non-adherent APCs resembled Transwell^{®}-derived APCs from primary endothelial cultures in surface marker phenotype pre- and post-stimulation with maturation factors (data not shown). Most importantly, over a series of donors, the T cell stimulatory capacity of APC derived from Transwell^{®}s that contained secondary HUVECs was comparable to other Transwell^{®}APCs and cytokine-derived DCs.

The results presented in these examples show that the Transwell^{®}-endothelial cell device provides a simple and quick approach to deriving DC-like cells from monocytes. The transmigrated monocytes morphologically resemble DCs and have increased expression of costimulatory molecules that are important in triggering complete T cell activation. Transwell^{®}-derived DCs are also very comparable to the well-characterized cytokine-derived DCs in generating T cell responses against *Candida albicans* in standard T cell assays. The advantages of the Transwell^{®} device, including the short incubation time required to get DC differentiation from monocytes, the modular design that allows for increasing system complexity that might make DCs more comparable to *in vivo* APCs, and its relatively low cost, make it an attractive alternative to current methods for generating DCs for *in vitro* experimentation.

## Claims

1. An *in vitro* method of evaluating the potential reaction of an animal to an agent, said method comprising:
- producing a first well comprising:
- a first porous membrane as the base;
- a ECM material affixed on top of said first porous membrane; and
- a second porous membrane affixed on top of said ECM material;
- inverting said first well into a second well comprising cell media;
- culturing endothelial cells on bottom of said first porous membrane;
- applying peripheral blood mononuclear cells (PBMCs) to the endothelial cells;
- after ~1.5 hours washing said PBMCs and said endothelial cells off of the bottom of said first porous membrane,
wherein dendritic cells are now present in said ECM material;
- removing said first well from said second well comprising cell media and placing said first well with said second porous membrane facing up into a third well comprising as its base a three-dimensional artificial lymphoid tissue, comprising a second ECM material and a plurality of lymphocytes and leukocytes;
- applying an agent to the top of said second porous membrane, allowing the dendritic cells to migrate out of said first ECM material and into said three-dimensional artificial lymphoid tissue; and
- *in vitro* evaluating the immune response to said agent.

2. The method of claim 1, wherein said first porous membrane and said second porous membrane are polycarbonate membranes.

3. The method of claim 1, wherein said first porous membrane and said second porous membrane have pores of ~5 µm.

4. The method of claim 1, wherein said agent is selected from the group consisting of a vaccine, an adjuvant, an immunotherapy candidate, an immunomodulator, a cosmetic, a drug, a biologic, a proinflammatory agent, and a chemical compound.

5. The method of claim 1, wherein the first porous membrane and the second porous membrane are laser-micromachined to increase porosity.

6. The method of claim 1, wherein said endothelial cells are human umbilical vein endothelial cells (HUVECs).

7. The method of claim 1, wherein said endothelial cells are a transformed endothelial cell line.

8. The method of claim 1, wherein said dendritic cells are CD14-positive.

9. The method of claim 1, wherein said porous membranes have pores of~5 µm

10. The method of claim 1, wherein said endothelial cells are cultured to confluency prior to adding the PBMCs, or wherein said endothelial cells are cultured until multilayer cell growth is achieved prior to adding the PBMCs.

## Patentansprüche

1. *In vitro*-Verfahren zur Bewertung der möglichen Reaktion eines Tiers auf ein Mittel, wobei das Verfahren umfasst:
- Vorbereiten einer ersten Vertiefung, umfassend:
- eine erste poröse Membran als die Grundlage;
- ein ECM-Material, angebracht auf der ersten porösen Membran; und
- eine zweite poröse Membran, angebracht auf dem ECM-Material;
- Invertieren der ersten Vertiefung in eine zweite Vertiefung, umfassend Zellmedien;
- Kultivieren von Endothelzellen am Boden der ersten porösen Membran;
- Aufbringen von peripheren mononukleären Blutzellen (PBMCs) auf die Endothelzellen;
- nach ~ 1,5 Stunden das Abwaschen der PBMCs und der Endothelzellen vom Boden der ersten porösen Membran,
wobei jetzt dendritische Zellen in dem ECM-Material vorhanden sind;
- Entfernen der ersten Vertiefung aus der zweiten Vertiefung, umfassend Zellmedien, und Platzieren der ersten Vertiefung mit der zweiten porösen Membran nach oben gerichtet in einer dritten Vertiefung, umfassend als ihre Grundlage ein dreidimensionales künstliches Lymphoidgewebe, umfassend ein zweites ECM-Material und eine Vielzahl von Lymphozyten und Leukozyten;
- Aufbringen eines Mittels auf die zweite poröse Membran, wobei das Mittel ermöglicht, dass die dendritischen Zellen aus dem ersten ECM-Material heraus und in das dreidimensionale künstliche Lymphoidgewebe hinein migrieren; und
- *in vitro*-Bewerten der Immunantwort auf das Mittel.

2. Verfahren nach Anspruch 1, wobei die erste poröse Membran und die zweite poröse Membran Polycarbonatmembranen sind.

3. Verfahren nach Anspruch 1, wobei die erste poröse Membran und die zweite poröse Membran Poren von ~ 5 µm aufweisen.

4. Verfahren nach Anspruch 1, wobei das Mittel aus der Gruppe ausgewählt ist, welche aus einem Vakzin, einem Adjuvans, einem potentiellen Immunotherapeutikum, einem Immunmodulator, einem Kosmetikum, einem Arzneistoff, einem biologischen Mittel, einem entzündungshemmenden Mittel und einer chemischen Verbindung besteht.

5. Verfahren nach Anspruch 1, wobei die erste poröse Membran und die zweite poröse Membran lasermikrobearbeitet werden, um die Porosität zu erhöhen.

6. Verfahren nach Anspruch 1, wobei die Endothelzellen humane Nabelvenenendothelzellen (HUVECs) sind.

7. Verfahren nach Anspruch 1, wobei die Endothelzellen eine transformierte Endothelzelllinie sind.

8. Verfahren nach Anspruch 1, wobei die dendritischen Zellen CD14-positiv sind.

9. Verfahren nach Anspruch 1, wobei die porösen Membranen Poren von ~ 5 µm aufweisen.

10. Verfahren nach Anspruch 1, wobei die Endothelzellen vor der Zugabe der PBMCs bis zu Konfluenz kultiviert werden, oder wobei die Endothelzellen vor der Zugabe der PBMCs kultiviert werden bis Mehrschichtzellwachstum erreicht ist.

## Revendications

1. Procédé *in vitro* d'évaluation de la réaction potentielle d'un animal à un agent, ledit procédé comprenant :
- la production d'un premier puits comprenant :
-- une première membrane poreuse comme base ;
-- un matériau ECM fixé sur le haut de ladite première membrane poreuse ; et
-- une seconde membrane poreuse fixée sur le haut dudit matériau ECM ;
- l'inversion dudit premier puits dans un second puits comprenant des milieux cellulaires ;
- la culture de cellules endothéliales sur le fond de ladite première membrane poreuse ;
- l'application de cellules mononucléaires sanguines périphériques (PBMC) aux cellules endothéliales ;
- après environ 1,5 heure, le lavage desdites PBMC et desdites cellules endothéliales hors du fond de ladite première membrane poreuse,
dans lequel les cellules dendritiques sont maintenant présentes dans ledit matériau ECM ;
- l'élimination dudit premier puits dudit second puits comprenant les milieux cellulaires et le placement dudit premier puits avec ladite seconde membrane poreuse orientée vers le haut dans un troisième puits comprenant, à sa base, un tissu lymphoïde artificiel en trois dimensions, comprenant un second matériau ECM et une pluralité de lymphocytes et de leucocytes ;
- l'application d'un agent sur le haut de ladite seconde membrane poreuse, permettant aux cellules dendritiques de migrer hors dudit premier matériau ECM et dans ledit tissu lymphoïde artificiel en trois dimensions ; et
- l'évaluation *in vitro* de la réponse immune audit agent.

2. Procédé selon la revendication 1, dans lequel ladite première membrane poreuse et ladite seconde membrane poreuse sont des membranes de polycarbonate.

3. Procédé selon la revendication 1, dans lequel ladite première membrane poreuse et ladite seconde membrane poreuse ont des pores d'environ 5 µm.

4. Procédé selon la revendication 1, dans lequel ledit agent est choisi dans le groupe constitué d'un vaccin, d'un adjuvant, d'un candidat à l'immunothérapie, d'un immuno-modulateur, d'un cosmétique, d'un médicament, d'un agent biologique, d'un agent pro-inflammatoire, et d'un composé chimique.

5. Procédé selon la revendication 1, dans lequel la première membrane poreuse et la seconde membrane poreuse sont micro-usinées au laser pour augmenter leur porosité.

6. Procédé selon la revendication 1, dans lequel lesdites cellules endothéliales sont des cellules endothéliales de veine ombilicale humaines (HUVEC).

7. Procédé selon la revendication 1, dans lequel lesdites cellules endothéliales sont une ligne de cellules endothéliales transformée.

8. Procédé selon la revendication 1, dans lequel lesdites cellules dendritiques sont positives pour CD 14.

9. Procédé selon la revendication 1, dans lequel lesdites membranes poreuses ont des pores d'environ 5 µm.

10. Procédé selon la revendication 1, dans lequel lesdites cellules endothéliales sont cultivées jusqu'à confluence avant d'ajouter les PBMC, ou dans lequel lesdites cellules endothéliales sont cultivées jusqu'à ce que la croissance cellulaire multicouches soit réalisée avant l'addition des PBMC.
